# EUROPEAN PATENT SPECIFICATION

(11) **EP 2 892 567 B1**
(45) Date of publication and mention of the grant of the patent: **21.02.2018**
(21) Application number: 13776578.0
(22) Date of filing: 04.09.2013
(51) Int. Cl.: A61K 47/50, A61K 38/18, C12N 15/87, G01N 33/50, C12Q 1/68

(54) **PARKINSON'S DISEASE TREATMENT BY BDNF-FLAG GENE TRANSFER THROUGH NEUROTENSIN POLYPLEX TO NIGRAL DOPAMINE NEURONS**
BEHANDLUNG VON MORBUS PARKINSON MITTELS BDNF-FLAG-GENTRANSFER DURCH DEN NEUROTENSINPOLYPLEX AN NIGRALE DOPAMINNEURONEN
TRAITEMENT DE LA MALADIE PARKINSON PAR UN TRANSFERT DE GÈNE DE DRAPEAU BDNF PAR L'INTERMÉDIAIRE DU POLYPLEXE DE NEUROTENSINE DANS DES NEURONES DOPAMINERGIQUES DE LA SUBSTANCE NOIRE

(30) Priority: 04.09.2012 MX PA12010244
(43) Date of publication of application: 15.07.2015
(73) Proprietor: Centro de Investigacion y de Estudios Avanzados del Instituto Politécnico Nacional, México, D.F. 07360 (MX); Martínez Fong, Daniel, 07300 México D.F. (MX)
(72) Inventor: MARTÍNEZ FONG, Daniel, 07300 México D.F. (MX)
(74) Representative: Germinario, Claudio
(86) International application number: PCT/IB2013/058274
(87) International publication number: WO 2014/037881

(56) References cited:
- WO-A2-2012/107908
- MARTINEZ-FONG DANIEL ET AL: "NTS-Polyplex: a potential nanocarrier for neurotrophic therapy of Parkinson's disease.", NANOMEDICINE : NANOTECHNOLOGY, BIOLOGY, AND MEDICINE OCT 2012, vol. 8, no. 7, October 2012 (2012-10), pages 1052-1069, XP55097190, ISSN: 1549-9642
- ARANGO-RODRIGUEZ M L ET AL: "Biophysical characteristics of neurotensin polyplex for in vitro and in vivo gene transfection", BIOCHIMICA ET BIOPHYSICA ACTA (BBA) - GENERAL SUBJECTS, ELSEVIER, AMSTERDAM, NL, vol. 1760, no. 7, 1 July 2006 (2006-07-01) , pages 1009-1020, XP025014910, ISSN: 0304-4165, DOI: 10.1016/J.BBAGEN.2006.02.021 [retrieved on 2006-07-01]
- GONZALEZ-BARRIOS ET AL: "Neurotensin polyplex as an efficient carrier for delivering the human GDNF gene into nigral dopamine neurons of hemiparkinsonian rats", MOLECULAR THERAPY, NATURE PUBLISHING GROUP, GB, vol. 14, no. 6, 18 November 2006 (2006-11-18), pages 857-865, XP005726589, ISSN: 1525-0016, DOI: 10.1016/J.YMTHE.2006.09.001

## Description

### Field of the invention.

The present invention pertains to the field of genomics and nanotechnology, specifically to *in vivo* transgene technology and its application in gene therapy. It consists of the performance of macromolecular vectors for transporting nucleic acids to neurons, involving neurotrophic therapy aimed at treating neurodegenerative diseases. Particularly, the present invention addresses the possibility of treating Parkinson's disease using a technology for transferring genes.

### Background of the invention.

Neurotrophins are structurally related proteins; they regulate the development and function of the central nervous system (CNS) and are essential for the survival of neurons, neurite outgrowth and synaptic plasticity (Huang and Reichardt, 2001). This family includes the nerve growth factor (NGF), the brain-derived trophic factor (BDNF), and neurotrophin 3, 4/5, 6, and 7. With the exception of NT-6, NT-7 found only in fish, other neurotrophins derive from the same ancestral gene phylogenetically found in chordates (Hallbrook et al., 2006). Neurotrophins are synthesized as pro-neurotrophins with an approximate weight of 30 kD and are cut to their mature form of approximate 14 kD in the cell compartment by the action of pro-hormone convertases enzymes such as furin (Seidah et al., 1996), or in the extracellular space by metalloproteinases 3, 7, and plasmin (Gray and Ellis, 2008). Mature neutrophins mainly form homodimers with an approximate weight of 30 kD and exert their action through binding to transmembrane receptors of the tyrosine kinase (TrK) receptors located in their target cells. Thus, TrKA is the high affinity receptor for NGF; TrKB for BDNF and NT-4/5, and TrKC for NT-3 (Murer et al., 2001; Numan et al., 2005). Ligand binding to the TrK receptor induces the homodimerization of TrK receptors and consequent autophosphorylation in their tyrosine kinase domains, creating docking sites for intracellular proteins. Activation of TrK receptors triggers signaling cascades such as phospholipase C (PLC-γ 1), Ras-MAPK (mitogen activated kinase), CREB (response element to the AMPc binding), and PI3K (phosphatidyl inositol-3 activated kinase) (Patapoutian and Reichardt, 2001; Skaper, 2012).

Notable among the functions induced by the binding of neurotrophins to their high-affinity specific receptors are the cell survival and differentiation, with axonal growth, dendritic remodeling, synapse formation, and protein expression crucial for normal neural function (Huang and Reichardt, 2001; Skaper, 2012). Aside from their binding to specific receptors, neurotrophins also possess a binding capacity to the low-affinity receptor for p75 neurotrophins, a family member of the receptors for tumor necrosis factor (TNF), which triggers the activation of signaling cascades that activate apoptosis (Koshimizu et al., 2010; Skaper, 2012).

Among the members of the neurotrophin family, BDNF is of particular importance from a therapeutic point of view since it has an enormous potential for neurotrophic therapy applied to Parkinson's disease (PD). This pathology is characterized by a chronic, degenerative, progressive and disabling result of the selective loss of dopaminergic neurons in substantia nigra pars compacta (CNS) and, consequently, the depletion of the neurotransmitter dopamine in the brain areas innervated by these neurons. As only one CNS dopaminergic neuron projects all basal ganglia involved in motor control and some areas of cerebral cortex, the neurodegeneration of such neurons gives rise to motor disorders and disorders of higher brain functions (Martinez-Fong et al., 2012). The etiology of PD is still under investigation, but it is known that several factors can lead to death of dopaminergic neurons producing the movement disorders observed in PD; this type is known as idiopathic Parkinson, Parkinsonism, or Parkinsonian syndrome. The threshold for clinically dopaminergic dysfunction is yet unknown; there is an assumption that motor alterations appear when there is a loss of 70% of the dopamine levels in the stratium and 50% of cellular death of dopaminergic neurons in CNS (Ugrumov et al., 2011). So, it has been suggested that the neurotrophic restorative therapy is the best strategy to treat Parkinsonism, as it is intended to prevent cellular death of the dopaminergic neurons surviving neurodegeneration and restoring functional innervation to the synaptic projection nucleus. The background of the present invention strongly supports the use of BDNF in restorative therapy for PD.

BDNF is described for the first time in the 1980s, 30 years after the identification of NGF as trophic factor in chicken embryos; but unlike NGF, BDNF was isolated from porcine brain homogenates (Bennet et al., 2002). Similarly to other neurotrophins, BDNF is synthesized as a 32 kD immature precursor, pro-BDNF. In the trans-Golgi network, BDNF is cut by enzymatic action to its mature form of 14 kD (Mowla et al., 2001). Both BDNF in its mature form and its immature precursor is included in secretory vesicles and released into the extracellular space. To exert its biological action, BDNF forms 28 kD dimers. BDNF and its TrKB receptor are widely distributed in the central nervous system in the hippocampus, amygdala, basal forebrain, bark, stem, cerebellum, and midbrain (Murer et al., 2001). It is important for the present invention that BDNF and its TrKB receptor are expressed particularly in CNS dopaminergic neurons from adult animals (Benisty et al., 1998; Numan and Seroogy, 1999; Seroogy et al., 1994) and humans, including Parkinsonian patients (Howells et al., 2000; Nishio et al., 1998; Parain et al., 1999).

BDNF levels in the CNS vary during the development of rats' central nervous system, and it is very likely that the same should happen in humans. At birth, the levels are low in rats, then increasing throughout postnatal development and declining again in adulthood (Numan et al., 2005; Zhou et al., 1996). Contrary to the changes in the BDNF levels, the expression of its TrKB receptor does not decrease in development or adulthood in the population of neurons positive for tyrosine hydroxylase (TH+) in the CNS (Numan et al., 2005). This finding strongly suggests that the dopaminergic neurons retain their ability to respond to BDNF brain maturity stage, despite the low levels of this neurotrophin in adulthood.

Because of its widespread distribution in the central nervous system, BDNF neurotrophic effects mediated by its TrKB receptor are exercised in a variety of neurons including dopaminergic neurons. In early studies by Barde, BDNF proved able to induce survival in cultures of spinal sensory neurons of chicken embryos (Barde et al., 1982). On this basis, BDNF has been associated with the promotion of neuronal survival in various neural populations such as retina ganglion cells (Johnson et al., 1986), cortical neurons (Ghosh et al., 1994), hippocampal (Lindholm et al., 1996; Lowenstein and Arsenault, 1996), cholinergic (Alderson et al., 1990; Ward and Hagg, 2000) y and dopaminergic (Baquet et al., 2005; Hyman et al., 1991).

It is highly relevant for the present invention to highlight the fact that the addition of human recombinant BDNF or purified BDNF from porcine brain in dissociated cultures of ventral midbrain of rat embryos E14-16 old promotes the survival of TH+ cells (Hyman et al., 1991). In co-cultures of slices of ventral and striatum midbrain obtained from newborn rats, it has also been demonstrated that the addition of BDNF significantly increases TH+ neurons and the density of their axons projecting to the co-cultured striatal brain slices (Ostergaard et al., 1996). These results are of particular significance for neurotrophic therapy by evidencing the development of a dual neurotrophic effect of BDNF on the dopaminergic neurons; on one hand, BDNF promotes neural survival, and on the other, it stimulates the axonal growth and regeneration. It is desirable that effective neurotrophic therapy for neurodegenerative disease is oriented to rescue dying neurons and in turn, stimulates the regeneration of the fibers projecting to different targets of innervation.

*In vitro* experiments highlight the protective role of BDNF against two of the selective neurotoxins for dopaminergic neurons that are widely used in developing animal models of PD. It has been demonstrated that primary cultures of midbrain dopaminergic neurons or SH-SY5Y cultured cells of neuroblastoma dopaminergic phenotype treated with BDNF and subsequently exposed to 6-OHDA or MPP+ had a lower rate of cellular death compared to control cultures (30% vs. 70%) (Hyman etal., 1991; Spina etal., 1992).

Evidence obtained from mice during the early postnatal stage show that BDNF prevents the cellular death of dopaminergic population of CNS. Studies in mice aged 5 to 6 demonstrated that the removal of BDNF using neutralizing antibodies induces the death of neurons in CNS (Oo et al., 2009). Similarly, blocking the production of BDNF by unilateral infusion of antisense oligonucleotide for 28 days in the CNS of adult rats also decreases in 40% the number of TH+ neurons and induces contralateral rotational behavior after systemic administration of apomorphine, which reflects the development of Hemiparkinsonism (Porritt et al., 2005). These findings suggest that BDNF plays a crucial role in the maintenance and operation of dopaminergic neurons in the brain of young animals.

However, studies in genetically engineered animals shed controversial results on the role of BDNF in the survival of dopaminergic neurons in CNS. In BDNFfl/fl Nestin-CRE mice, the elimination of BDNF in the brain during postnatal development did not affect the number of dopaminergic neurons in adulthood (Oo et al., 2009). Similar results were obtained in mice deprived of BDNF during early postnatal development (BDNF-/-) and in mice with chronically reduced levels to 50% during adulthood (BDNF-/+). An interesting fact found in these mice is that dopaminergic neurons had few dendrites. These results suggest that BDNF affects the maturation of these neurons, but not their survival (Baker et al., 2005). By contrast, studies in Wnt-BDNFKO mice lacking selectively BDNF in the midbrain and cerebellum, showed a 23% decrease in the number of TH+ neurons in the persistent CNS until postnatal day 120 (Baquet et al., 2005). Moreover, transgenic animals showed nigral architectural changes and alterations in Parkinsonian type motor behavior (Baquet et al., 2005).

An opposite effect to the reduction in the number of TH+ neurons found in animals lacking selectively BDNF in the midbrain and hindbrain (Baquet et al., 2005) was reported by a study in DBH: BDNF transgenic mice, which over expressed BDNF in noradrenergic and adrenergic neurons because of the BDNF gene fusion to the promoter of dopamine beta-hydroxylase gene (Alonso-Vanegas et al., 1999). In these mice, the total number of nigral dopamine neurons was 52% higher than in control animals (Alonso-Vanegas et al., 1999). The results of both sets of studies support the conclusion that BDNF can prevent the death of dopaminergic population or promote neuronal survival. Generally, the results of *in vitro* studies conducted during the development and in genetically engineered animals support the proposal that BDNF, owed to its neurotrophic action on dopaminergic neurons in the CNS, may be used in the treatment of PD.

Injecting the neurotoxin 6-hydroxydopamine (6-OHDA) into the nigrostriatal system is one of the selective lesion models most used for dopaminergic neurons so far as it mimics most of the motor alterations of PD, and its effects are in line with what happens in the nigrostriatal system mechanical lesion. The administration of 6-OHDA in the dopaminergic system has shown to increase de level of BDNF protein in the striatum and CNS two weeks after the lesion (Zhou et al., 1996). These findings suggest that the increased expression of BDNF is part of the physiological response to repair the damage in the nigrostriatal system, although this spontaneous restorative response is unable to achieve a functional repair of the system.

Several research groups using axotomy medial forebrain bundle to damage the nigrostriatal dopaminergic system have also documented increased levels of mRNA for BDNF and/or TrKB after the lesion, suggesting the possibility of the development of spontaneous recovery from damage. It has been described that increased expression levels of both molecules persist until two weeks after the section of the medial forebrain bundle (Garcia Navia et al., 2008; Venero et al., 2000). Furthermore, Garcia Navia et. al. (2008) showed sprouting of dopaminergic fibers in the CNS and in the lesion site three months after axotomy of the medial forebrain bundle. However, plastic changes by the increase of the binomial BDNF - endogenous TrKB were insufficient to restore the function of the damaged nigrostriatal dopaminergic system. These findings point to the need to over-express BDNF and/or TrKB to induce a more efficient neurotrophic effect in the lesioned nigrostriatal system accompanied by improvements in motor behavior.

Various preventive and restorative strategies have been assayed to provide exogenous BDNF *in vivo* models of the nigrostriatal system lesion in face of the failure of spontaneous neurotrophic response to repair the damaged dopaminergic system.

A preventive strategy is to produce increased levels of BDNF before practicing the nigrostriatal dopaminergic system lesion in experimental animals. It has been demonstrated that infusion of BDNF on the dopaminergic system prior to mechanical damage by axotomy (Hagg, 1998) or an increased endogenous BDNF striatal lesion induced by kainate (Canudas et al., 2005) partially prevent the death of dopaminergic neurons in the CNS. Other experimental approaches have tested the neuroprotective effect of BDNF transplants by using embryonic dopaminergic neurons in hemiparkinsonian rats. It was found in these studies that pretreatment of cultures with BDNF increases the functionality of the implant reflected by the decrease in rotational behavior induced by amphetamine three weeks post transplant (Zhou et al., 1997). Furthermore, the intra ventricular administration of BDNF (Sauer et al., 1993), or into the culture transplanted (Yurek et al., 1996) promotes survival of TH+ neurons in the transplantation and the reduction in the rotational behavior induced by amphetamine. Reducing the rotational behavior reflects the relief of Hemiparkinsonism as explained in detail below.

The preventive strategy has also been evaluated with *ex vivo* gene therapy and *in vivo* gene therapy with the aim of providing a single application procedure of BDNF at constant levels to the lesioned nigrostriatal dopaminergic system. The *ex vivo* gene therapy has been tested using fibroblasts genetically modified to produce BDNF, which were transplanted into the striatum or CNS of adult guests prior to the damage of the dopaminergic system by 6-OHDA or MPP+ (Frim et al., 1994; Levivier et al., 1995). Both cases showed that implantation of fibroblasts producing BDNF prior to the striatal administration of 6-OHDA or MPP+ partially prevents denervation and death of nigral dopaminergic neurons induced by these neurotoxins (Frim et al., 1994; Levivier et al., 1995). To date *in vivo* gene therapy has been tried only using viral vectors to perform the transfer of the BDF transgene into the central nervous system of experimental animals prior to the lesion induced by 6-OHDA. A research group used recombinant adeno-associated viral vector to produce bicistronically BDNF-myc and green fluorescent protein, which was injected into the CNS of adult rats six months prior to the unilateral lesion with 6-OHDA. Although transgenic BDNF-myc had no effect on the number of dopaminergic neurons in the CNS, it indeed prevented the development of Hemiparkinsonism, which was measured by the rotational behavior induced by amphetamine 10 weeks after the lesion (Klein et al., 1999). Similar results were obtained with the viral vector based on the Herpes Simplex virus capable of transducing BDNF (HSV-1). Here, the HSV-1 vector was injected into the striatum induced 4 weeks before inducing Hemiparkinsonism with 6-OHDA. Ten weeks after the lesion, the animals which received the gene therapy with BDNF gene showed a reduction in apomorphine-induced rotation, but the expression of BDNF exerted a poorly protective effect against the cytotoxicity of 6-OHDA in dopaminergic neurons (Sun et al., 2005). As with rotational behavior induced by amphetamine, the reduction of the rotational behavior induced by apomorphine reflects the relief of Hemiparkinsonism as explained in detail below.

In summary, these findings suggest that BDNF, despite its source, protects the nigrostratial system if its administration is prior to the damage. Although neurotrophic preventive therapy is successful in experimental animals, it is unlikely to be brought into clinical practice owed to the lack of diagnostic tools to accurately predict the time of occurrence of dopaminergic neurodegeneration in PD.

Restorative strategy is aimed at producing an increase in BDNF levels time after the nigrostriatal dopaminergic system has been lesioned in experimental animals. Currently, there are scarce research works studying the regenerative effect of BDNF after neurotoxic damage. In this line, astrocytes genetically engineered to secrete BDNF have been used to transplant them into the striatum lesion 15 days after the induction of Hemiparkinsonism with 6-OHDA. In this work, BDNF reduced the amphetamine-induced rotational behavior by 45%, one and a half months after transplantation (Yoshimoto et al., 1995). Recently, the restoring effect of BDNF was confirmed using mesenchymal stem cells of human origin capable of releasing BDNF by epigenetic induction (Somoza et al., 2010). The transplantation of these cells in the ipsilateral CNS of hemiparkinsonian rats 1 week after striatal injection of 6-OHDA produced significant hypertrophy of TH+ nigral cells, an increase immunoactivity for TH in the striatum, and the stabilization of amphetamine-induced motor symptoms (Somoza et al., 2010). Despite the potential of BDNF in neurotrophic therapy for PD, to date there are no studies using BDNF gene transfer in restorative protocols.

In the background of the present invention, we emphasize the crucial role of BDNF in the maintenance and survival of dopaminergic neurons in the adult brain. Therefore, when dopaminergic neurons suffer a degenerative process as in PD, they lose intrinsic neurothrophic support (BDNF), which exacerbates the neurodegenerative process. In this context, it has been documented that mRNA and BDNF protein are reduced to the couple of the loss of dopaminergic neurons in the CNS of PD patients, which underlines that neurons still surviving can express BDNF (Howells et al., 2000; Parain et al., 1999). Consequently, a therapy aimed at increasing levels of BDNF on the CNS dopaminergic neurons that have survived the degenerative process appears to be the most logical approach in treating PD. Post-mortem hybridization examinations *in situ* on parkinsonian patients show that the TrKB levels in CNS decrease concomitantly to the loss of dopaminergic neurons; they note however that the remaining neurons showed no difference in the mRNA level expressed regarding the neurons of control brains (Benisty et al., 1998). The fact that dopaminergic neurons surviving the degenerative process do express TrKB strongly suggests that these cells retain their ability to respond to BDNF stimulation and therefore predicts the neurotrophic therapy success with BDNF in PD.

Looking towards the development of neurotrophic alternatives for PD treatment, in our laboratory we have developed a tool for gene transfer to cells possessing and internalizing the neurotensin (NTS) type 1 (NTSR1) receptor (Arango-Rodriguez et al., 2006; Martinez-Fong et al., 1999; Rubio-Zapata et al., 2009). This technological development known as NTS polyplex consists of biodegradable nanoparticles resulting from the compaction of DNA plasmid by electrostatic binding of caryophillic peptide (PK), derived from protein capsid Vp1 of SV40 virus, and the "NTS carrier." The latter is a conjugate between the poly-L-lysine with NTS and fusogenic peptide HA2 of influenza virus hemagglutinin (PF). This complex is disclosed in Patents MX264932 (Martinez-Fong D, 2009) and MX287089 (Martinez-Fong D, 2011). Several studies have shown that dopaminergic neurons of CNS express and internalize NTSR1 (Castel et al., 1994; Nouel et al., 1997; Palacios and Kuhar, 1981; Szigethy and Beaudet, 1989). Because of this feature, NTS-polyplex can efficiently and selectively transfer genes to dopaminergic neurons of CNS in experimental animals (Alvarez-Maya et al., 2001; Gonzalez-Barrios et al., 2006; Navarro-Quiroga et al., 2002). The NTS-polyplex ability to transfect to CNS dopaminergic neurons *in vivo* is of particular relevance to the object of the present invention.

The results of this study point to the potential application of BDNF as a therapeutic strategy for PD, but using new strategies for obtaining its targeted and controlled expression; the present invention reveals the biochemical, morphological, and behavioral recovery in hemiparkinsonian rats induced by gen BDNF-flag transference to dopaminergic neurons through the nanoparticle system NTS-polyplex.

In the prior art approach, this is described as double blind controlled clinical trial, which has proved the feasibility of gene therapy for Parkinson's disease; it was performed by using an adeno-associated vector type 2 (AAV2) to transfect the neurturin gene in both trophic putamens of PD patients in advanced stage. The rational basis for the use of AAV2-neurturin vector was that it would be internalized by striatal neurons, which then would express and secrete neurturin, and the released neurturin would be then retrogradely transported through the remaining fibers in degeneration to the cell body of the CNS (Marks et al., 2010). Unfortunately, the assessment of the patients 12 months after transfection showed no significant improvement in their motility for the primary motility evident in patients receiving placebo surgery. However, a subset of AAV2-neurturin-transfected patients evaluated 18 months after surgery showed modest beneficial effects, but significantly compared to control individuals. As the authors state, a possible explanation for the lack of significant motor recovery 12 months after transfection with AAV2-neurturin could be that the transgene was sent in the advanced stages of PD, when the degenerative course of the disease has reached the point where only a few dopaminergic axons remained in the putamens to collect and transport the neurturin. According to this latter possibility, the same research team reported that the putamen of 2 patients transfected with AAV2-neurturin showed immunoreactivity for neuturin (approx 15%) on par scattered TH induction. However, little evidence of neurturin and no increase in TH immunoreactivity were found in the CNS (Marks et al., 2010). In contrast, one year after the AAV2-neurturin transfection in the caudate putamen of Rhesus monkeys showing a dopaminergic innervation integrated in the nigrostriatal system, a TH-increased immunoactivity was observed and the hypertrophy of TH+ cells in the substantia nigra (Herzog et al., 2009). Based on preclinical and clinical experience obtained with the transfection of AAV2-neurturin, the authors consider justifiable the search of a therapy targeting mainly the dopaminergic neurons of the CNS.

### Brief description of the figures.

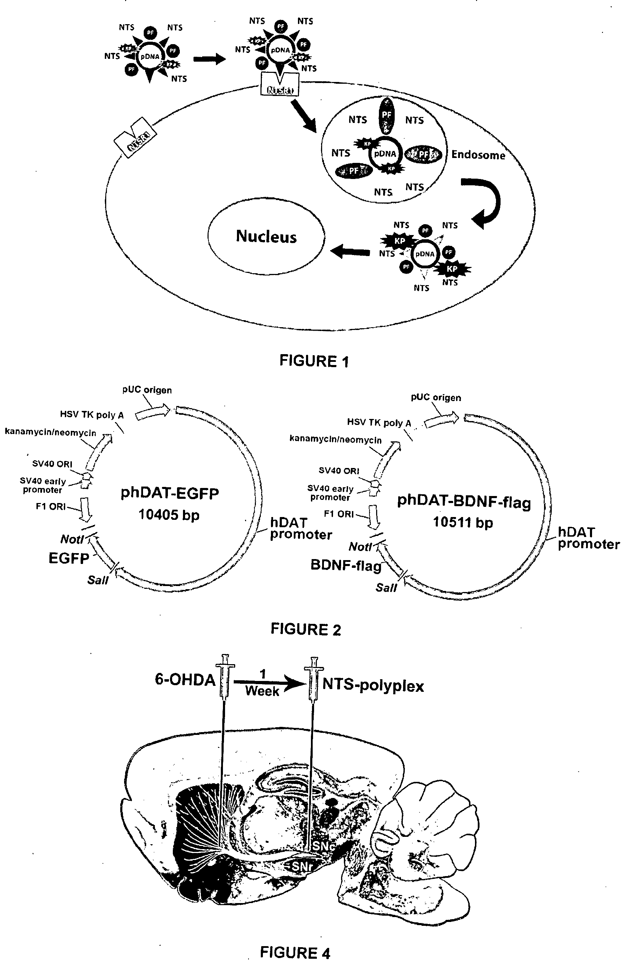

**Figure 1**. Shows the mechanism of gene transfer via NTS-polyplex. Here, we can observe fusogenic peptide (PF), caryophillic peptide (PK), neurotensin (NTS), and NTS type 1 receptor (NTSR1).
**Figure 2****.** Shows a simplified diagram of the plasmids phDAT-EGFP-N1 (10,405 bp) and phDAT-BDNF-flag (10,511 bp) coding for green fluorescent protein (GFP), and the brain-derived neurotrophic factor with the flag label (BDNF-flag), respectively, under the control of regulatory sequences from the gene promoter for human dopamine transporter (hDAT). The scheme emphasizes the large size of the hDAT promoter in proportion to the other components of the plasmid and the cleavage sites for the restriction enzymes Not1 and Sal1.

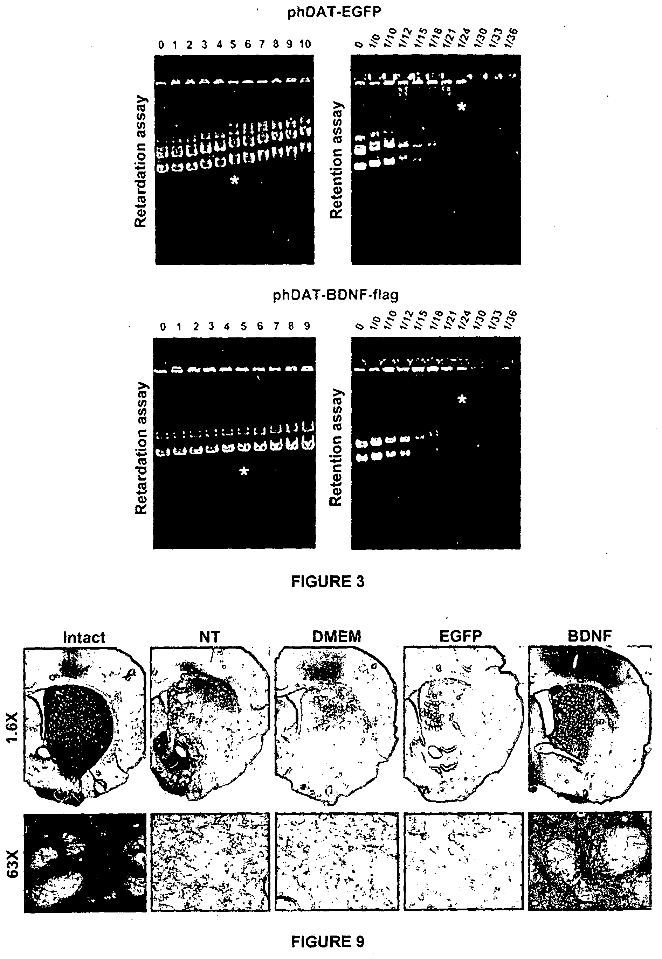

**Figure 3****.** Shows photographs of 0.8% agarose gels showing the electrophoretic pattern of DNA-caryophillic peptide complexes (retardation assay) and NTS-polyplex (retention assay) formed at increasing molar ratios. In all cases, 0 = single DNA plasmid (6 nM). Numbers on the retardation gels correspond to micro molar concentrations of caryophillic peptide (PK), which are incubated with a constant concentration of DNA plasmid (6 nM) to form the DNA-PK. The ratios on the retention gels correspond to different polyplex formed with a constant part of the DNA-PK complex (6 nM : 5 µM) and increasing parts of neurotensin carrier (a conjugate of poly-L-lysine, NTS, and PF) at nano molar concentrations with respect to the plasmid DNA. The asterisks correspond to optimal molar ratios of DNA: PK (retardation assay) and of the complex DNA-PK: NTS carrier to form NTS-polyplex (retention assay). The optimal molar ratios to form NTS-polyplex were 6 nM plasmid DNA (phDAT-EGFP or phDAT-BDNF-flag), 5 µM PK and 144 nM NTS carrier (ratio 1:24).
**Figure 4****.** Shows a diagram illustrating the injection sites of 6-OHDA and transfection with NTS polyplex. To generate hemiparkinsonian rats, 6-OHDA (20 µg/3 µL of saline solution containing 1% ascorbic acid) in one of the striations according to the following coordinates: anteroposterior (AP), 0 mm from the Bregma; mediolateral (ML) + 4 mm; dorsoventral (DV), 5.2 mm from the dura. One week after the lesion, 3 µL of NTS-polyplex is injected in the ipsilateral substantia nigra according to the following coordinates: AP, -5.4 mm from the Bregma; ML, +1.5 mm, DV -6.8 mm from the dura. The coordinates are set to male Wistar rats weighing 220 g.

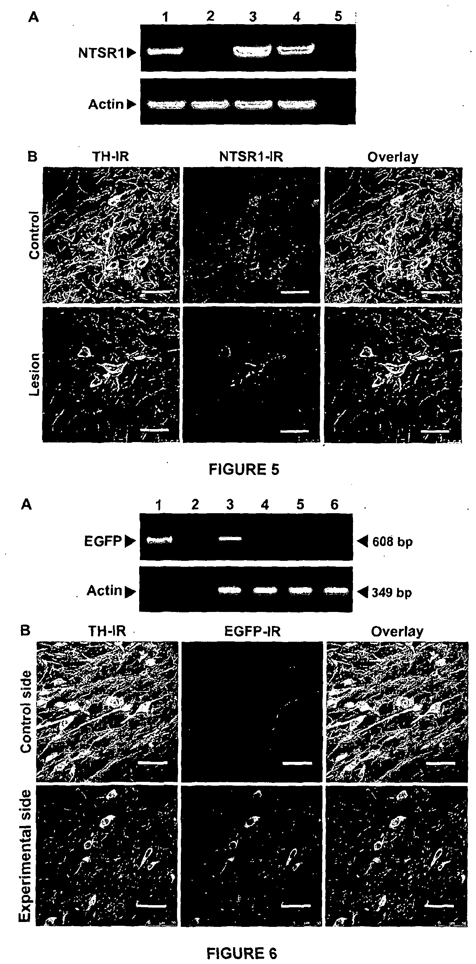

**Figure 5****.** Shows the persistence of the expression of neurotensin receptor type 1 (NTSR1) in tyrosine hydroxylase (TH) positive neurons of the substantia nigra 1 week after lesion caused with 6-OHDA. Shows (A) a representative photograph of an agarose gel showing the electrophoretic band of amplicon NTSR1 after reverse transcription and polymerase chain reaction assay (RT-PCR). Actin was used as constitutive gene. Lane 1, N1E-115 cells (positive control); lane 2, L929 cells (negative control); lane 3, healthy substantia nigra; lane 4, lesioned substantia nigra; lane 5, PCR reagents without cDNA (internal negative control); and (B) representative confocal micrographs of double immunofluorescense assay against TH and NTSR1 in the midbrain of a hemiparkinsonian rat. The micrographs correspond to the substantia nigra of the normal side (control) and the 6-OHDA lesioned side of the same rat. Scale = 25 µm.
**Figure 6****.** Shows the expression of green fluorescent protein (GFP) in tyrosine hydroxylase (TH) positive neurons in the substantia nigra of a hemiparkinsonian rat two weeks after transfecting phDAT-EGFP plasmid using NTS-polyplex. Shows (A) representative photograph of a 5% polyacrylamide gel showing the GFP amplicon electrophoretic band after the RT-PCR assay. Actine was used as constitutive gen. Lane 1, phDAT-EGFP plasmid (positive control); lane 2, PCR reagents without cDNA (internal negative control); lane 3, substantia nigra lesioned with 6-OHDA and transfected with phDAT-BDNF-flag; lane 4, healthy substantia nigra without transfection; lane 5, lesioned and transfected side of ipsilateral striatum; lane 6, healthy striatum without transfection; and (B) representative confocal micrographs of double immunofluorescence assay against TH and GFP in the midbrain of hemiparkinsonian rats. The micrographs correspond to the substantia nigra of the normal side (control side) and the lesioned and transfected side (experimental side) of the same rat. The green signal represents TH immunoreactivity revealed by the fluorescent secondary antibody, while the red signal represents the immunoreactivity of the green fluorescent protein (GFP) as revealed by rhodaminated secondary antibody. Scale bar = 50 µm.

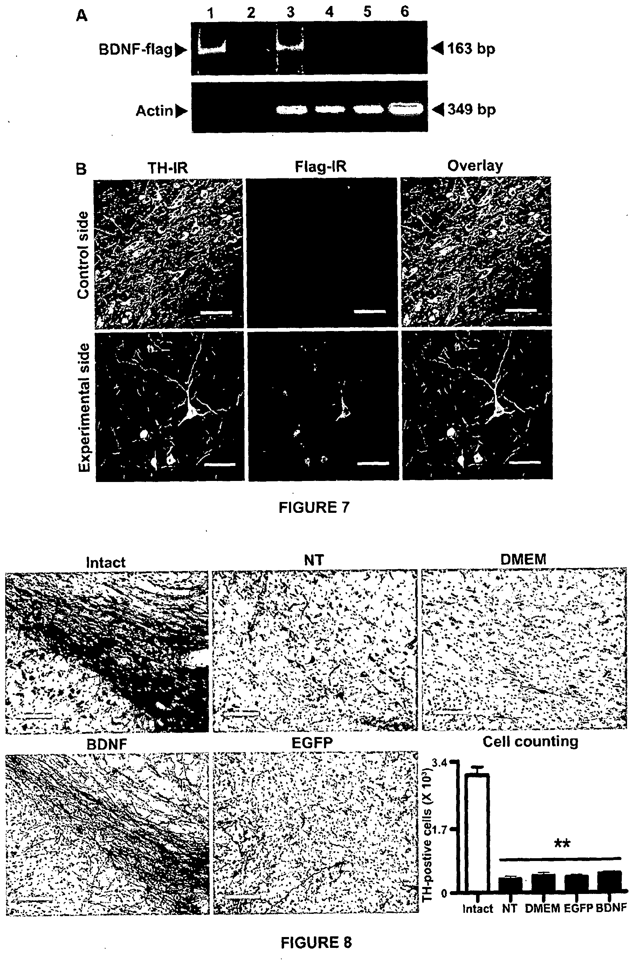

**Figure 7****.** Shows the expression of BDNF-flag in the nigral dopaminergic neurons of a hemiparkinsonian rat 2 weeks after transfecting phDAT-BDNF-flag plasmid using NTS-polyplex. Shows (A) representative photography of BDNF-flag amplicon separated by agarose gel after RT-PCR assay. Actin was used as constitutive gene. Lane 1, phDAT-BDNF-flag plasmid (positive control); lane 2, PCR reagents without cDNA (internal negative control); lane 3, substantia nigra lesioned with 6-OHDA and transfected with phDAT-BDNF-flag; lane 4, healthy substantia nigra without transfection; lane 5, lesioned and transfected side of ipsilateral striatum; lane 6, healthy striatum without transfection; and (B) representative confocal micrographs of double immunofluorescence assay against tyrosine hydroxylase (TH) and flag in the midbrain of a hemiparkinsonian rat. Micrographs correspond to the substantia nigra of the normal side (control side) and the lesioned and transfected side (experimental side) of the same rat. Scale bar = 50 µm.
**Figure 8****.** Shows the restoration of TH+ fibers in the substantia nigra of a hemiparkinsonian rat 2 weeks after transfection with phDAT-BDNF-flag plasmid using NTS-polyplex. Micrographs representing immunohistochemistry against tyrosine hydroxylase (TH) and counterstained with cresyl violet in the substantia nigra of hemiparkinsonian rats three weeks after the 6-OHDA striatal lesion and 2 weeks after applying one of the following treatments to the ipsilateral substantia nigra: no transfection (NT); Dubelcco's Modified Eagle's Medium (DMEM) injection; phDAT-EGFP (GFP) or phDAT-BDNF-flag (BDNF) transfection. "Intact" refers to a healthy substantia nigra. The graph (inset) shows the TH+ cell count in the experimental conditions listed above. Values are expressed as mean ± SE; *n* = 3 animals per group. ** *P* < 0.001 vs. the intact side. Scale = 100 µm.
**Figure 9****.** Shows the remarkable recovery of TH+ fibers in the striatum of a hemiparkinsonian rat 2 weeks after phDAT-BDNF-flag using NTS polyplex. Representative micrographs of immunohistochemistry against tyrosine hydroxylase (TH) in the stratium of hemiparkinsonian rats 3 weeks after 6-OHDA striatal lesion and 2 weeks after applying one of the following treatments to the ipsilateral substantia nigra: no transfection (NT); Dubelcco's Modified Eagle's Medium (DMEM) injection; phDAT-EGFP (GFP) or phDAT-BDNF-flag (BDNF) transfection. "Intact" refers to a healthy substantia nigra. The values on the left correspond to fold amplification.

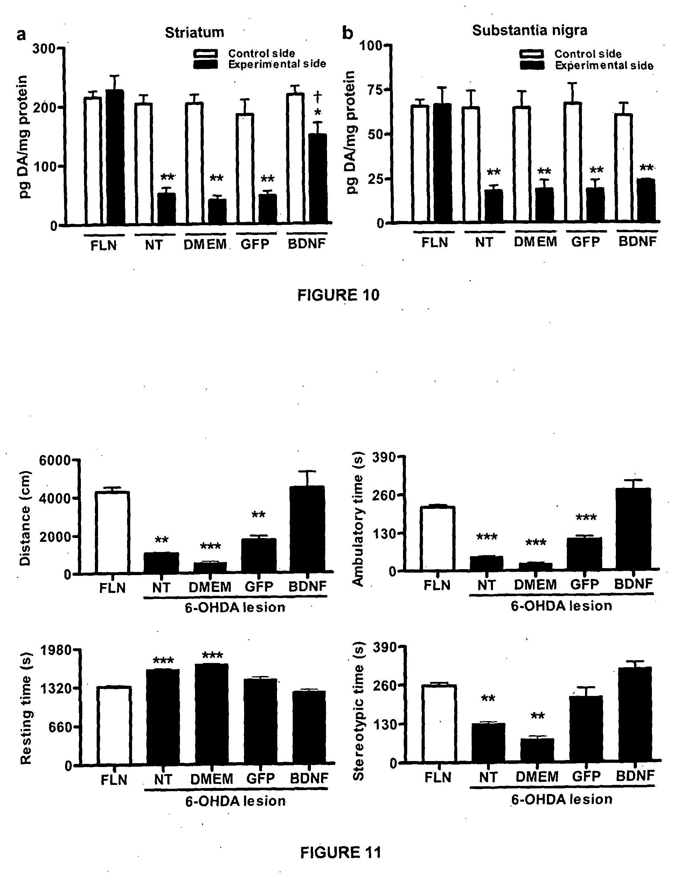

**Figure 10****.** Shows the partial recovery of striatal dopamine levels 2 weeks after BDNF-flag gene transfection in dopaminergic neurons using NTS-polyplex. Dopamine levels in the striatum (A) and the substantia nigra (B) were measured using HPLC with electrochemical detection 3 weeks after the 6-OHDA unilateral micro injection into a stratium and 2 weeks after applying one of the following treatments to the ipsilateral substantia nigra: no transfection (NT); Dubelcco's Modified Eagle's Medium (DMEM) injection; phDAT-EGFP (GFP) or phDAT-BDNF-flag (BDNF) transfection. The false lesion group (FLN) was injected into the striatum with PBS (6-OHDA vehicle). The control side refers to healthy tissues (striatum or substantia nigra) contralateral to the side of the lesion and transfection (experimental side). Values are expressed as mean ± SE; *n* = 8 animals per group. * *P* < 0.05, ** *P* < 0.001 vs. the values from the false lesion group (FLN). † *P* < 0.05 vs. the values from the side without transfection (NT).
**Figure 11****.** Shows the spontaneous motor behavior of hemiparkinsonian rats in open field, 2 weeks after transfecting the substantia nigra using NTS-polyplex. FLN refers to the group of rats with a false lesion in the striatum. One week after striatal injection of 6-OHDA, treatments were applied to the substantia nigra as follows: no transfection (NT); Dubelcco's Modified Eagle's Medium (DMEM) injection; phDAT-EGFP (GFP) or phDAT-BDNF-flag (BDNF) transfection. Values are expressed as mean ± SE; *n* = 8 animals per group. * *P* < 0.05, ** *P* < 0.001, *** *P* < 0.001 vs. the group of false lesion (FLN) (one-way ANOVA followed by Bonferroni's test).

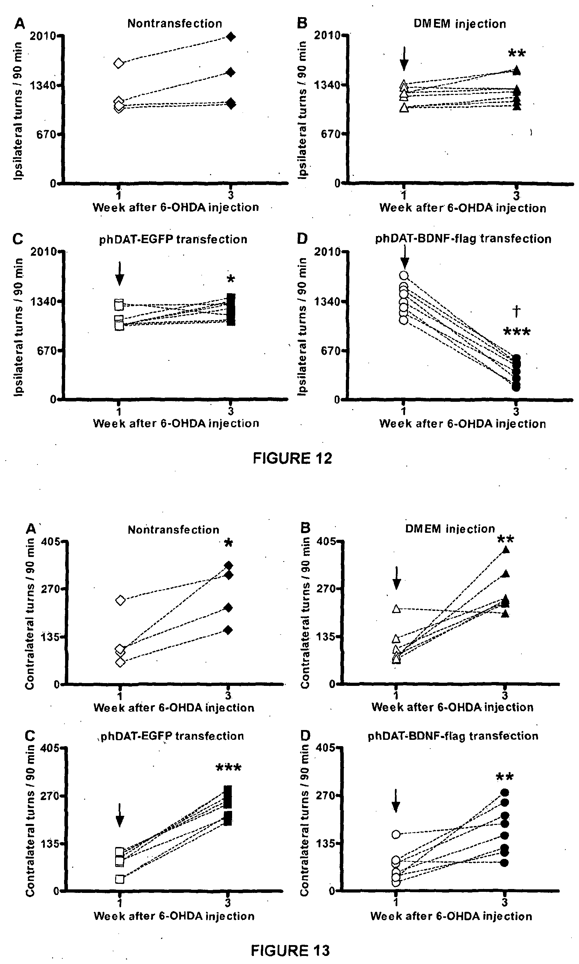

**Figure 12****.** Shows the amphetamine-induced rotation behavior in hemiparkinsonian rats 2 weeks after transfection of the substantia nigra using NTS-polyplex. One week after the striatal injection of 6-OHDA, following treatments were applied to the substantia nigra (arrow): (A) no transfection; (B) Dubelcco's Modified Eagle's Medium (DMEM) injection; (C) phDAT-EGFP (GFP) or (D) phDAT-BDNF-flag (BDNF) transfection. Ipsilateral rotation behavior was induced by amphetamine (8 mg/Kg, i.p.) at weeks 1 and 3 after the 6-OHDA striatal lesion. Values are expressed as mean ± SE; *n* = 8 animals per group. * *P* < 0.05, ** *P* < 0.01, *** *P* < 0.001 vs. the values measured on week 1 (paired test). † *P* < 0.05 vs. the values of the other groups on week 3 (ANOVA followed by Bonferroni's test).
**Figure 13****.** Shows the apomorphine-induced rotation behavior in hemiparkinsonian rats, two weeks after transfection of the substantia nigra using NTS-polyplex. One week after striatal injection of 6-OHDA, the following treatments were applied to the substantia nigra (arrow): (A) no transfection; (B) Dubelcco's Modified Eagle's Medium (DMEM) injection; (C) phDAT-EGFP (GFP) or (D) phDAT-BDNF-flag (BDNF) transfection. Contralateral rotational behavior was induced by apomorphine (0.5 mg/Kg, s.c.) at weeks 1 and 3 after the 6-OHDA stratial lesion. Values are expressed as mean ± SE; *n* = 8 animals per group. * *P* < 0.05, ** *P* < 0.01, *** *P* < 0.001 vs. the values measured in week 1 (paired test). † *P* < 0.05 vs. the values of the other groups on week 3 (ANOVA followed by Bonferroni's test).

### Detailed description of the invention.

The present disclosure proposes the use of a gene transfer technology that is based on receptor-mediated endocytosis on the cell surface and involves the use of the referred NTS-polyplex, which is capable of being endocytosed by the receptor denominated NTSR1, in the treatment of PD as described below.

For purposes of the present disclosure, we used as neurotrophic-effect DNA the phDAT-BDNF-flag under the control of the regulatory sequences from the gene promoter for the human dopamine carrier (hDAT), which confers additional specificity transfection of the dopaminergic neurons and a prolonged expression of the transgene.

The NTS-polyplex system consists of biodegradable nanoparticles resulting from the compaction of plasmid DNA resulting from the electrostatic binding of PK and the NTS carrier. As mentioned above, PK is the nuclear translocation domain of the capsid protein Vp1 of the SV40 virus, whereas the NTS carrier is a conjugate of poly-L-lysine, NTS, and PF. PF is the HA2 fusogenic domain of hemagglutinin of the influenza virus (Martinez-Fong et al., 2012). Figure 1 shows the contribution of each component of NTS-polyplex gene transfer through NTSR1 internalization. In NTS-polyplex, the NTS acts similarly to the endogenous ligand, so that, upon binding to NTS, the NTSR1 induces endocytosis of this receptor carrying the NTS-polyplex. Once in the endosome, the acid pH produced by the hydrogen pumps causes the PF to adopt an alpha-helix conformation, which alters the permeability of the endosomal membrane, enabling the NTS-polyplex to escape from endosome. In the cytoplasm, the plasmid DNA is guided into the cell nucleus by PK intervention by possibly using the basic importins system. Finally, the transgene in the plasmid DNA is transcribed and translated in the encoded protein to trigger the adequate biological activity (Martinez-Fong et al., 2012). The use of hDAT promoter to control the expression of the transgene confers the NTS-polyplex a new advantage; aside of providing a second point of specificity mediated by NTS-polyplex, it restricts the transgenic expression to the dopaminergic neurons, favoring the prolonged expression of the transgene as described below (figure 1).

Previous studies in the lab have demonstrated the efficiency of the NTS-polyplex to transfer the glial-derived neurotrophic factor (GDNF) gene to dopaminergic neurons of animals with marked Hemiparkinsonism symptoms (Gonzalez-Barrios et al., 2006). In this study, the GDNF transferred by the NTS-polyplex to previously damaged neurons could rescue the dopaminergic neurons in degeneration process, maintain the nigrostriatal innervation, and reduce motor impairment induced by a 6-OHDA lesion (Gonzalez-Barrios et al., 2006). The literature on the neuroprotective action of GDNF as a powerful protector of dopaminergic neurons has been confirmed by several studies on the lesion of the nigrostratial system. However, GDNF is not produced physiologically by the dopaminergic neurons, but primarily by the surrounding astrocytic population (Airaksinen and Saarma, 2002). Furthermore, the GDNF-transgene expression with NTS-polyplex that Gonzalez Barrios (2006) used in his work, is controlled by the human elongation factor alpha1 promoter. This promoter, while useful for basic studies, has the disadvantage of showing a very potent transcriptional activity, therefore producing excessive levels of the transgene of interest. Hence, its use for regulating the expression of neurotrophic factors in clinical practice may be counterproductive, since it has demonstrated adverse effects by the excessive production of GDNF and BDNF (Georgievska et al., 2002; Sajadi et al., 2005). Furthermore, as constitutive in all cells of the human body, this promoter shows a transcriptional activity that lacks specificity (Wakabayashi-Ito and Nagata, 1994). For an effective and safe anti-parkinsonian therapy, it is desirable for the expression of the transgene to be constrained to the neurotrophic-affected neurons and to produce adequate levels of neurotrophic protein. In the present disclosure, we assessed two novel aspects of NTS-polyplex. First, a BDNF gene is used, which is an intrinsic neurotrophic factor for dopaminergic neurons. Therefore, one of the approaches of the present disclosure is to reposition the same neurotrophic factor lost by dopaminergic neurons during the degenerative process of PD, and which they also need to perform physiological functions aside from its known neurotrophic effects (Bosse et al., 2012; Madara and Levine, 2008; Tucker and Fadool, 2002). Secondly, hDAT promoter is used to command the expression of the transgene under the physiological control of the transcriptional mechanisms that are characteristic of the dopaminergic neurons. Therefore, another approach of the present disclosure is to further restrain the transgenic expression of the dopaminergic neurons and steadily provide physiological levels of BDNF transgenic protein. These advantages are described in the present disclosure using an animal model that resembles a moderate to severe stage of neurodegeneration in PD.

During the experimental work underlying the present disclosure, we achieved that the transfer of BDNF-flag gen through NTS-polyplex will cause the expression of said transgene under the control of hDAT promoter in the remaining dopaminergic neurons after the lesion. After this achievement, we succeeded in promoting neuronal survival, inducing the reinnervation to target nuclei (primarily striated), concomitantly restoring the dopamine levels, and reducing the altered motor behavior. To accomplish all this, which is evident from our results, we had to consider optimal experimental conditions, addressing the validity of the experimental model of PD, assessing and identifying the biological effects of the transfected BDNF as we faced the possibility of not obtaining the minimum expression levels that proved necessary or excessive. There was also the possibility of inducing neurogenesis and/or reinnervation.

In the present disclosure, we had to overcome five technical difficulties to accomplish the results presented here, and which constitute the support for the proof of concept concerning the use of NTS-polyplex in treating PD.

The first technical difficulty was distinguishing the expression of transgenic BDNF from that of endogenous BDNF as dopaminergic neurons of animals and humans express it physiologically (Howells et al., 2000; Nishio et al., 1998; Parain et al., 1999; Seroogy et al., 1994); also its sequence is highly conserved between mammals (Maisonpierre et al., 1991). To overcome this difficulty we used the BDNF coding sequence of the same species in which we would transfect it; i.e., a rat, but containing the flag label that is a nucleotide sequence encoding a small artificial peptide (Asp-Tyr-Lys-Asp-Asp-Asp-Asp-Lys). Thus, the transgenic expression can be identified at mRNA by the RT-PCR level and at protein level by immunostaining techniques. The RT-PCR results of the present disclosure using specific oligonucleotides to identify a Flag and the whole BDNF demonstrate the mRNA expression for BDNF-flag in the CNS after transfection with the NTS-polyplex (figure 7A). In this sense, the results of double immunofluorescence against flag and TH show the presence of flag immunoreactivity in TH+ neurons (figure 7B). These results suggest that the BDNF-flag construction is useful to show the expression of the transgenic protein in the dopaminergic neurons. In clinical tests, the BDNF-flag construction will be most useful to correlate the transgenic expression identified in *postmortem* studies with the PD improvement documented in the medical record, and thus validate the effectiveness of gene therapy with BDNF.

The second technical difficulty was to achieve that the presence of Flag would not affect the physiological function of BDNF. Generally, the fusion peptides such as Flag have no physiological effect *per* se, but may interfere with the biological function of the protein of interest if the fusion alters the receptor-binding domain. Bioinformatics studies suggested that the Flag could bind to the amino or carboxyl terminus of BDNF without interfering with the binding domain for receptor TrkB as internal addition would affect the functional structure of BDNF. In the present disclosure, we used the Flag sequence united to the 3' end of the BDNF coding sequence of the rat. The results presented here demonstrate that only the transfected rats with pAD1-BDNF-flag via the NTS-polyplex showed morphological recovery evidences of the nigrostriatal system (figures 8 and 9), increased levels of dopamine in the striatum (figure 10A), and a significant decrease in behavioral disturbances (figures 11, 12, and 13). Together, these results show that the BDNF-flag protein is still biologically active, and they support its usefulness in clinical assays with Parkinson patients.

The third technical difficulty was to achieve that dopaminergic neurons would express effective and secure levels of BDNF-flag. It is widely known that the expression levels of a transgenic protein depend on the activity of the transcriptional promoter under which it is subcloned into the plasmid. The elongation alpha 1 promoter, which has a very powerful regulatory activity (Suarez et al., 2006), is not the ideal candidate to direct the expression of BDNF-flag transgene, as it would lead to an excessive production of BDNF-flag protein, originating harmful side effects (Georgievska et al., 2002; Sajadi et al., 2005). Another undesirable possibility produced by excessive levels of BDNF is the activation of p75 receptor and consequently, the development of apoptosis (Bamji et. al., 1998; Casaccia-Bonnefil et al., 1999). Furthermore, the activity of the elongation alpha 1 promoter is nonspecific, being constitutive in all eukaryotic cells (Suarez et al., 2006; Wakabayashi-Ito and Nagata, 1994). Likely, viral promoters such as cytomegalovirus (CMV) or the early promoter of the simian virus (SV40) are not ideal candidates for directing the expression of BDNF-flag as they are very powerful and in addition, have the disadvantage of being rapidly inactivated in eukaryotic cells by methylation processes (Brooks et al., 2004; Bryans et al., 1992). Experience has shown that CMV promoter produces transitory levels of transgenic protein in dopaminergic neurons transfected with the NTS-polyplex (Alvarez-Maya et al., 2001). Dopamine transporter (DAT) is a protein located in the pre synaptic membrane of the dopaminergic terminals responsible for eliminating released dopamine from the synaptic cleft, by that controlling the amplitude and duration of the dopamine signaling (Bannon, 2005). So, the promoter regulating the expression of the DAT gene is specific for dopaminergic neurons and their activity is regulated according to the physiological demands of DAT, which are minor compared to the demands of protein synthesis that is responsible for determining the activity of the elongation factor alpha 1 promoter (Bannon and Whitty, 1997; Bannon, 2005; Sacchetti et al., 1999; Suarez et al., 2006; Wakabayashi-Ito and Nagata, 1994). On this basis, we obtained in the present invention the plasmid construction denominated phDAT-BDNF-flag, wherein the promoter of the human DAT gene, to which we shall refer as hDAT promoter, regulates the expression of BDNF-flag. Of interest for use in gene therapy for PD, the results of the present disclosure demonstrate that hDAT promoter is functional in lesioned dopaminergic neurons after transfection with NTS-polyplex. In the present disclosure, we used phDAT-EGFP plasmid encoding the green fluorescent protein (GFP) under transcriptional control of hDAT promoter as positive control. The results of RT-PCR show the expression of mRNA for GFP (figure 6A) and the double immunofluorescence results against GFP and TH show the presence of GFP in TH+ cells (figure 7B). Consistent with these results, phDAT-BDNF-flag led to the expression of mRNA for BDNF-flag and translation of the corresponding protein in TH+ cells in the CNS lesioned with 6-OHDA (figure 7A). In addition for the specificity of transgene expression conferred by the promoter hDAT, the results demonstrate that the promoter is active during the dopaminergic neurodegeneration process induced by 6-OHDA, as the transgenic expression is clearly present two weeks after the transfection (figures 6 and 7), long enough to achieve reversal of Hemiparkinsonism motor alterations (figures 11, 12, and 13). It is possible that the activity of hDAT promoter to regulate the expression of BDNF-flag transfected by NTS-polyplex in the surviving neurons in parkinsonian patients achieve more effective therapeutic effects, which have not been achieved to date in the clinic by injecting viral vectors possessing powerful hybrid promoters (CMV amplifier and the promoter for chicken β-actin) to express the therapeutic protein, such as the case of neurturin (Marks et al., 2010; Ramaswamy et al., 2009).

The fourth technical difficulty was to achieve compaction of a plasmid as large as the phDAT-BDNF-flag (10.511 Kbp) in NTS-polyplex nanoparticles that were efficiently endocytosed by NTSR1 in dopaminergic neurons lesioned by 6-OHDA. The magnitude of transgenic material represents a difficulty for all vectors including viral vectors, which have a lower transport capacity than cationic polymers. The gel-electrophoretic retardation analysis of various combinations between the different components of the NTS-polyplex allowed determining the optimal molar ratio for achieving NTS-polyplex functional nanoparticles containing phDAT-BDNF-flag or phDAT-EGFP. The optimal molar ratios of these NTS-polyplex components were 6 nM plasmid, 5 µM PK, 144 nM NTS carrier, and 1% of inactivated fetal bovine serum. The total amount of injected plasmid was 555 ng / 3 µL for phDAT-BDNF-flag and 631 ng/3 µL for phDAT-EGFP, at a flow rate of 0.25 µL/min.

The fifth technical difficulty was to generate a lesion of the nigrostriatal dopaminergic system in the medium term after application of a single dose of 6-OHDA to reflect an intermediate to sever Parkinsonism, avoiding a mechanical lesion on the dopaminergic neurons of the CNS. Striatal injection of a single dose of 6-OHDA, known as "the Sauer model," produces a moderate lesion of the nigrostriatal dopaminergic system that could favor spontaneous regression of abnormal Parkinsonism (Sauer and Oertel, 1994). The "modified Sauer model," which consists of injecting four or more doses of 6-OHDA in different places of the dorsal striatum, has the drawback that the mechanical process of injection lesions the resident striatal neurons, so that this model may mask the behavioral evidence of nigrostriatal system recovery (Kirik et al., 1998). Finally, the injection of a single dose of 6-OHDA in the medial forebrain bundle has the advantage of producing a severe lesion to the nigrostratial system, but the disadvantage is that the lesion is acute and lesions the mesolimbic dopamine system, which also circulates through the same beam. Therefore, this model does not generate a pure Parkinsonism (Costall et al., 1976). The solution found in the present disclosure to validate the neurotrophic effect of BDNF-flag was injecting a single dose of 6-OHDA (20 µg/3 µL of saline solution) in the site where the medial forebrain bundle enters the striatum. At this site the mesolimbic system fibers are scarce, because they diverge before to innervate the acumbens nucleus, which is located in the ventral striatum. The coordinates used were: AP, 0 mm; ML + 4 mm, DV, 5.2 mm, taking the DV (dorsoventral) coordinate from dura and the AP (anteroposterior) coordinate from Bregma (figure 4). Under these conditions, the unilateral injection of 6-OHDA 20 µg/3 µL of saline solution) reduced 70 to 80% the TH+ population in the CNS (figure 8), and the density of TH+ fibers in the striatum (figure 9), and dopamine content in the CNS and the striatum lesioned side (figure 10A), one week after injection of the neurotoxin. Here appeared the motor symptoms of Hemiparkinsonism shown by ipsilateral rotation behavior induced by amphetamine (figure 12), and contralateral induced by apomorphine (figure 13). These behaviors appear because the unilateral lesion causes a biochemical and functional imbalance of the nigrostratial system. One hand, there is a less amount of dopamine in the nigrostriatal-system lesioned side compared to the normal side. Therefore, when injecting amphetamine, a drug that stimulates the release of dopamine, a greater amount of dopamine is released from the normal side than from the lesioned side and so, there is more motor activity on the normal side. This causes the rat to move rotating on the side of less motor activity (ipsilateral rotation to the lesion). Moreover, the absence of dopamine in the lesioned nigrostriatal-system causes hypersensitivity of the postsynaptic dopaminergic receptors in the striatum nucleus of the lesioned side. So, when injecting apomorphine, which is a dopamine agonist, the hypersensitized postsynaptic receptors are more stimulated than the normal receptors on the normal side. Here, a greater motor activity is generated in the lesioned side compared to the healthy side; then, when the rat moves, it tends to rotate to the opposite side of the lesion (contralateral rotation). These rotational-behaviors inducing by drugs are valuable tools for assessing the therapeutic effect of anti-Parkinson drugs or approaches. When these therapies are effective, there is a significant reduction in rotational behavior thus reflecting an improvement of Parkinsonism. A decreased rotational behavior induced by amphetamine is a result of correcting the dopamine levels in the lesioned side, whereas the decrease in rotational behavior induced by apomorphine is owed to decreased postsynaptic-receptor hypersensitivity on the lesioned side because of the correction of the dopamine levels on that side. The results of the present disclosure show that the rotational behavior induced by amphetamine only significantly decreased in Hemiparkinsonian animals transfected with BDNF-flag (figure 12). These results suggest that the dopamine levels increased in the lesioned side as shown by HPLC measurements (figure 10A). Rotational behavior induced by apomorphine was stabilized in Hemiparkinsonian animals within two weeks after transfection with BDNF-flag compared to lesioned control animals (figure 13), suggesting that the recovered dopamine levels stopped the advance of the hypersensitivity of the postsynaptic receptors. The present disclosure provides means for use in a therapeutic alternative for PD, which is the second most common neurodegenerative disease after Alzheimer's disease and a growing public health problem in Mexico; it is one of the leading causes of outpatient care at third level and has a large number of annual consultations owed to the chronicity of the disease and its specific management needs. In the coming decades, it is expected that PD patients will increase by the increase in life expectancy in Mexico (SSA). PD appears at a median age from 43 to 66 years (Diederich et al., 2003), affecting approximately 1-2% of subjects over 65 years (Vanitallie, 2008), and is more common in men than in women (Ramirez-Jirano et al., 2007).

Our results show that residual dopaminergic neurons of the CNS maintain the NTSR1 expression, supporting the idea that the NTS-polyplex accessed the dopaminergic neurons through the internalization of NTSR1 to deliver its genetic cargo into the cell nucleus and express the BDNF-flag protein. A cornerstone of the present disclosure is that the NTSR1 expression is not only maintained in experimental neurodegeneration (Goulet et al., 1999; Masuo et al., 1990), but also in PD patients, on whom the presence of mRNA and NTSR1 protein on nigral dopamine neurons has been clearly demonstrated by ligand-receptor binding techniques (Sadoul et al., 1984), auto radiography (Uhl et al., 1984), and *in situ* hybridization (Yamada and Richelson, 1995). These findings support the possibility of using the NTS-polyplex to transfect the BDNF gene in survivor dopaminergic neurons from early to intermediate stages of PD.

The assertion that BDNF exerts its effect on dopaminergic neurons through an autocrine or paracrine mechanism has been supported by findings of co-expression of the BDNF and TrkB receptor (Numan and Seroogy, 1999; Seroogy et al., 1994). In PD, the reduced levels of BDNF and TrkB accompanying the progressive death of dopaminergic neurons inevitably lead to the loss of the one source of endogenous trophic factor to stop the progress of neurodegeneration (Howells et al., 2000; Parain et al., 1999). Our results in the experimental model of PD show that by strengthening the source of the endogenous trophic factor via the transfection of the surviving neurons, achieves the rescue of the functional innervation of the nigrostriatal system.

We demonstrated that the expression of the phDAT-BDNF-flag plasmid caused a robust arborization of dopaminergic fibers in the CNS and in the striatum (figures 8 and 9), and increased the dopamine levels in the striated nucleus previously lesioned with 6-OHDA (figure 10A) in the early period of 2 weeks after transfection. However, although there was also a significant arborization in the transfected CNS, the number of dopaminergic neurons neither increased nor the dopamine levels in the nucleus (figures 8 and 10B).

These findings suggest that the neurogenesis was not induced by the expression of phDAT-BDNF-flag, which is consistent with literature reports where BDNF is transfected to the CNS via a viral vector (Klein et al., 1999). Despite this fact, the partial recovery of striatal dopaminergic innervation associated with the expression of BDNF-flag led to a substantial reversal of motor asymmetry in Hemiparkinsonian rats, shown by a decrease of 70% in the number of amphetamine-induced rotations, and 50% of apomorphine-induced rotations vs. the control group (figures 12 and 13).

The fact that the expression of phDAT-BDNF-flag prevented completely the motor alterations in the spontaneous movement in Hemiparkinsonian animals is even more notable (figure 11). It is possible that the behavioral improvement is due to the partial recovery of the innervation in the striatum, known to be stopped the hypersensitivity development in dopaminergic receptors (Gonzalez-Barrios et al., 2006) as suggested by the results of the present disclosure (figures 9 and 10A).

Morphological and functional recovery of acute Hemiparkinsonism in the rat indicates that the dopaminergic neurons retained their ability to respond to BDNF and strongly suggests the presence of the TrkB receptor in these neurons.

Based on the evidence of mRNA expression and the TrkB protein in the CNS of Hemiparkinsonian patients (Benisty et al., 1998), our findings point to the possibility of a successful therapy with BDNF using the NTS-polyplex in the early or intermediate stages of PD.

Recently, the therapy based on a neurturin transgene sent by an AAV to putamen cells of human parkinsonian patients did not return any clinically significant benefit because of a poor retrograde transport of neurturin transgene bodies of dopaminergic neurons of the CNS (Marks et al., 2010). Based on this treatment failure, the authors of that work have decided to conduct a new study aimed to dopaminergic cells of the CNS (Marks et al., 2010). The results herein show that the NTS-polyplex fully covers the ideal criterion as gene transporter specifically targeted to the dopaminergic cells of the CNS, as it induced the expression of BDNF-flag only in the dopaminergic neurons surviving the 6-OHDA lesion; an expression that was accompanied by the evident arborization of TH+ fibers and the significant restoration of the dopamine content in the striatum ipsilateral to the lesion, and the significant decrease of the spontaneous motor and rotation behavior induced by drugs. From this evidence, we propose that the NTS-polyplex is a potential vector for the transfection of phDAT-BDNF-flag to dopaminergic neurons of patients in early to intermediate stages of PD.

A clear advantage of using NTS-polyplex lies in the fact that it would only be internalized by dopaminergic neurons that express NTSR1 selectively, in contrast to the viral vectors that are captured by all cell lines surrounding the injection site (Bartus et al., 2011). Furthermore, based on proven evidence that the NTS-polyplex is able to co-transfect two reporter genes *in vitro* (Arango-Rodriguez et al., 2006), it is possible that this capacity could be highlighted if used for simultaneous sending two or more genes of various neurotrophic molecules. Another alternative could be the co-transfection of BDNF and TrkB.

Although preclinical studies have allowed gaining an insight on the potential benefit of using gene therapy to restore dopaminergic innervation in PD, there is also evidence of the limitations of this procedure. For example, those protocols where GDNF was expressed have been associated to undesirable side effects resulting from an aberrant innervation of the DA fibers in regeneration (Georgievska et al., 2002), and the decrease in dopamine synthesis (Sajadi et al., 2005). Other studies have documented the striatal dopaminergic hypofunction after chronic intranigral administration of BDNF (Lapchak et al., 1993). These issues concerning safety emphasize the need for better vectors and transgenes that provide maximum benefits with minimal adverse effects. For example, regulation of gene expression should be implemented in gene vectors so that it is possible to switch off gene expression in the event of the development of unexpected adverse effects. Notwithstanding possible limitations, the object of the invention disclosed herein, is at present to propose that the NTS-polyplex as claimed is a vector for the transfection of phDAT-BDNF-flag to dopaminergic neurons of patients in early to intermediate states of PD, and we present the necessary experimental tests.

In conclusion, the results of our work coupled with the success of preclinical studies with other transgenes for neurotrophic factors strongly support the feasibility of using the NTS-polyplex to provide an efficient and selective recovery of striatal dopaminergic innervation in the early and intermediate stages of PD.

The basic condition to enable gene transfer via the NTS-polyplex is for the cell population to possess NTSR1 (a high affinity receptor for neurotensin) (Alvarez-Maya et al., 2001; Martinez-Fong et al., 1999; Martinez-Fong et al., 2012; Navarro-Quiroga et al., 2002). The demonstration of the NTSR1 expression in the dopaminergic neurons under severe hemiparkinsonism conditions is the cornerstone of this work. The first result was to show that the dopaminergic neurons originally expressing NTSR1 retain this receptor 8 days after 6-OHDA striatal insult. Upon histological evaluation with double immunofluorescence, confocal microscopy images revealed that the remaining nigral neurons that are immunoreactive to TH are also immunoreactive to NSTR1, by that demonstrating that they retain the NTSR1. This first evidence is shown in figure 5B where the immunoreactivity to TH is shown in the green channel (TH-IR); the immunoreactivity denoting the presence of NTSR1 is shown in the red channel (NTSR1-IR); and the co-localization of both signals is shown as the yellow color of the superimposed image. It should be noted that all TH-immunoreactive neurons have NTSR1, both in control conditions and in hemiparkinsonism conditions. We found that NTSR1 is present in the soma and dendritic processes in the CNS intact and lesioned. Moreover, electrophoretic analysis of RT-PCR studies performed on samples of the CNS shows the band corresponding to the NSTR1 amplicon transcript on the lesioned and control sides (figure 5A). This result demonstrates the expression of mRNA for NTSR1 and reinforces double immunofluorescence results. NTSR1 presence (figure 5) makes the surviving dopaminergic neurons to neurotoxic damage candidates to be transfected with the NTS-polyplex.

After verifying the presence of NTSR1 in neurons of Hemiparkinsonian animals after 1 week evolution, but with frank symptoms of dopaminergic imbalance, we proceeded to the transfection with NTS-polyplex with the phDAT-EGFP reporter plasmid or hDAT-BDNF-flag plasmid.

Two weeks after randomly assigning animals with extensive lesion to transfection with either plasmid, we evaluated the expression and the presence of GFP and BDNF-flag transgenic proteins. To evaluate the expression of both proteins, we used the RT-PCR technique in samples of the CNS of the control side and the side lesioned and transfected (experimental side) using specific oligonucleotides for both cases. Evidence of the amplification of GFP transcript and BDNF-flag transcript is shown in figures 6A and 7A respectively. Panel A of figure 6 shows the band of 608 bp separated by electrophoresis corresponding to GFP transcript only amplified in the CNS on the transfected side (lane 3). Similarly, panel A of figure 7 shows the band of 163 bp corresponding to the BDNF-flag transcript only amplified in the CNS on the transfected side (lane 3). Lane 1 shows the amplicon of GFP (figure 6A) and BDNF-flag (figure 7A) of the corresponding plasmid used as positive control of PCR technique. These results show at mRNA level the expression of both transgenes transfected by the NTS-polyplex.

To show the cellular localization of the non-neurotrophic GFP reporter protein in the remaining dopaminergic neurons, we evaluated their expression by the technique of double immunofluorescence against TH, revealed with a fluorescent antibody, and GFP revealed with a rhodaminated antibody in Hemiparkinsonian rats. The analysis of images taken with confocal microscopy revealed that two weeks after transfection with phDAT-EGFP-N1 plasmid, the immunoreactivity of the transgenic protein (GFP-IR) was located in TH immunoreactive neurons (TH-IR) of the CNS transfected and lesioned (experimental side), and was absent in the untransfected side of the CNS (figure 6).

To distinguish transgenic BDNF from endogenous BDNF in the remaining dopaminergic neurons, we performed double immunofluorescences against the flag label and the TH. Panel B of figure 7 shows the normal population of TH+ neurons in the intact CNS (control side) and the significant decrease both in the number of TH+ cells and their dendritic processes in the CNS 3 weeks after the 6-OHDA lesion (experimental side). Interestingly, the immunoreactivity of Flag (Flag-IR) was located only in few TH+ neurons of the lesioned CNS, which were transfected with phDAT-BDNF-flag (experimental side). These results and the result of RT-PCR lead to the conclusion that the dopaminergic neurons surviving the 6-OHDA lesion were able to express BDNF-flag, 2 weeks after transfection with NTS-polyplex.

After having demonstrated the specific BDNF-flag expression in the dopaminergic neurons lesioned with 6-OHDA and transfected with phDAT-flag using the NTS-polyplex, we proceeded to demonstrate the neurotrophic effect of BDNF-flag at morphological, biochemical, and behavioral level as described below.

The data obtained from immunohistochemistry against TH in visible light showed that 3 weeks after intrastriatal administration of 6-OHDA the dopaminergic neurons ipsilateral to the side of the lesion suffered retrograde degeneration, which is evidenced by the decrease in the number of TH+ cells vs. the contralateral intact CNS (figure 8). Concomitant with the loss in the number of TH+ neurons, we observed a significant decrease in the TH+ arborization in the CNS (figure 8). To demonstrate the neurotrophic effect, we transfected ipsilaterally the phDAT-flag with the NTS-polyplex in the CNS one week after the 6-OHDA lesion and evaluated the number of neurons and the density of the TH+ arborization 2 weeks after the transfection. The negative controls used were the transfection of the GFP non-neurotrophic transgene, the injection of the NTS-polyplex vehicle (DMEM), and the absence of nigral transfection (NT). The results show that the animals transfected with phDAT-BDNF-flag developed a robust arborization of TH+ fibers in the CNS transfected vs. the negative control animals, but the density was lower than that of the normal CNS (figure 8). When performing counts of TH+ neurons in each condition, we found that all experimental conditions showed a significant decrease (P < 0.001) of 80% vs. the population of the contralateral CNS in the same brains (figure 8). These results demonstrate that the short-term effect of BDNF-flag is exerted on the neuritic tree and not on the neurogenesis.

In parallel, we analyzed the striata of the same groups of animals that were subjected to immunohistochemistry studies against TH in the CNS. As in this nucleus, the intrastriatal injection of 6-OHDA produced a drastic decrease in the immunoreactivity to TH in the striatum, suggesting that there was extensive dopamine denervation in the striatum owed to the death of neurons in the CNS (figure 9). This striatal denervation remains unchanged in no transfection conditions (NT), false transfection with the NTS-polyplex vehicle (DMEM), and the transfection with phDAT-GFP-N1 (GFP). In contrast, the previously lesioned striata, ipsilateral to the CNS transfected with phDAT-BDNF-flag (BDNF), showed a significant recovery of immunoreactivity to TH vs. the striated nuclei of the negative controls animals. These results suggest that the expression of BDNF-flag induced the recovery of the dopaminergic innervation to the striatum (figure 9).

A portion of the animals (n = 8 per group) subjected to behavioral assessment of each experimental group was assigned to the biochemical assessment of the dopamine levels in the CNS and the striatum by high-performance liquid chromatography (HPLC). The results show that the dopamine level in the CNS in all experimental conditions showed a significant reduction of 70% vs. their intact counterparts, 3 weeks after the ipsilateral striatum lesion (figure 10A). Similarly, striatal dopamine decreased approximately 70% vs. the intact contralateral striata, 2 weeks after the transfection with GFP, the false transfection with DMEM, or the lack of transfection, as shown in figure 10B. In contrast, the dopamine level found in the striata of hemiparkinsonian animals transfected with the BDNF-flag of the present disclosure recovered 75% of the total dopamine content determined on their intact counterparts (figure 10B).

Consistent with morphological and neurochemical recovery an improvement was also registered in spontaneous motor behavior assessed in the open field (figure 11). Two weeks after transfection with the BDNF-flag of the present disclosure, the hemiparkinsonian animals significantly increased the total distance traveled in the 30 minutes assessment vs. the groups receiving no transfection or transfected with a reporter gene (figure 11A). Hemiparkinsonian animals treated with BDNF-flag also showed higher values than those obtained in untransfected hemiparkinsonian groups or transfected with GFP in ambulatory movement time and stereotypic movement; these values did not show significant differences vs. the control group (figures 11B and 11D). Additionally to the increase of the mobility time, hemiparkinsonian animals treated with BDNF-flag remained less time at rest than the non-transfected animals (NT) and the false transfection animals (DMEM) (figure 11C). In summary, the animals treated with the BDNF-flag of the disclosure showed no statistically significant differences in any of the parameters evaluated in the spontaneous mobility test in the open field vs. the false-lesion group (FLN). We conclude then that there was a behavioral recovery owed to the treatment with BDNF-flag.

The assessment of the rotational behavior induced by amphetamine, indirect dopaminergic agonist, behaviorally determines the changes in balance among the dopaminergic systems of both hemispheres; so that their administration causes the rat to move rotating on its axis to the lesioned side. This is an indicator that the dopaminergic system ipsilateral to the direction of rotation has a lower dopamine level than the healthy contralateral dopaminergic system. In this experiment the rotation induced by amphetamine was evaluated 8 days after the lesion, functioning as a selection criterion for nigral transfection, and 2 weeks after the treatment with phDAT-BDNF-flag, with phDAT-EGFP-N1 (GFP), the false transfection with DMEM or in absence of nigral transfection (NT), which reflects the natural progression of the lesion. For statistical purposes, we compared the number of rotations made by each animal before and after each nigral transfection treatment. The results obtained are shown in figure 12; wherein only the animals receiving the transfection of BDNF-flag neurotrophic gene showed a significant reduction of 72% in the number of rotations induced by amphetamine to the number of rotations during the condition before the transfection (figure 12D). The decrease in rotational behavior induced by amphetamine in hemiparkinsonian animals transfected with BDNF-flag reflects the dopamine recovery in the lesioned side, as shown by HPLC, and it is therefore, a clear evidence of decreased hemiparkinsonism.

The rotational behavior stimulated by apomorphine, dopamine agonist, is also a tool to assess the activity imbalance of both dopaminergic nigrostriatal systems caused by hemiparkinsonism. In the denervated striatum, a hypersensitivity of the dopaminergic receptors is developed owed to the depletion of dopamine and thus, they are more sensitive to dopamine agonists stimulus compared to the normal contralateral striatum. Therefore, the administration of apomorphine rouses more activity in the lesioned side and the rat to move rotates on its axis to the healthy side (contralateral rotation). This experiment was performed 24 hours after the experiment of rotation induced by amphetamine, 9 days after striatal lesion with 6-OHDA, and 2 weeks (+2 days) after intranigral transfection with plasmids phDAT-EGFP-N1 (GFP), phDAT-BDNF-flag (BDNF), the false transfection applying only the vehicle of NTS-polyplex (DMEM), or in absence of transfection (NT), which reflects the evolution of the striatal lesion (figure 13). Under these experimental conditions, the average number of contralateral rotations induced by apomorphine one week after the 6-OHDA lesion in hemiparkinsonian animals was 95 ± 25 rotations in 90 min. While 2 weeks after intranigral transfection the average of contralateral rotations in control hemiparkinsonian animals was 255 ± 5 rotations in 90 min, which represents an increase of 168% vs. the number or rotations performed before the transfection (figure 13C). By contrast, the group of hemiparkinsonian rats transfected with BDNF-flag developed 161 ± 32 ipsilateral rotations in 90 min, which is 63% lower (P < 0.05) than the rotations shown by the control hemiparkinsonian groups (figure 13D). The decrease in the rotation behavior induced by apomorphine in hemiparkinsonian animals transfected with BDNF-flag reflects the decrease of the hypersensibility of the dopaminergic postsynaptic receptors on the lesioned side owed to the dopamine recovery and is further evidence of reduced hemiparkinsonism.

The values of the histological and behavioral assessments were analyzed with *t*-Student parametric tests for repeated measures to compare the differences between the same animal before and after each treatment, and analysis of variance (ANOVA) of one-way for independent groups to compare the differences between experimental groups. The graphs and statistical tests were performed with the software GraphPad Prism 4 (San Diego, CA, USA), and results were expressed using the arithmetic mean (X) and standard error (SE). To detect differences between groups, we used Bonferroni *post hoc* tests. The accepted significance level was *P* < 0.05 for *t*-Student, ANOVAs and *post hoc* tests.

The main objective of the present disclosure is to provide a non-viral strategy based on the use of the nanoparticles denominated neurotensin polyplex (NTS-polyplex) for the targeted delivery of the BDNF transgene to dopaminergic neurons of the substantia nigra to treat PD; it comprises as embodiment of the disclosure the possibility of preventing the symptoms of this disease, reduce the symptoms or parkinsonism once they have arisen, and/or reverse the neurodegenerative process in patients of any stage of PD with striatal dopamine levels decreased up to 80%, while there still remains a minimal percentage of dopaminergic neurons in the substantia nigra, preferably at an early stage where there still are 50 to 60% of neurons in the substantia nigra and 20% dopaminergic innervation in the putamen.

Another objective of the present disclosure relates to the use of neurotensin polyplex (NTS-polyplex) to transfect the surviving dopaminergic neurons of the substantia nigra in PD patients at early to intermediate stages of the disease and thus, to restore the BDNF neurotrophin expression with subsequent reversal of the symptoms of PD or parkinsonism.

Within the embodiments of the present invention is to provide the elements for use of a neurotensin polyplex or NTS-polyplex as claimed in the context of the disclosure.

It is set as embodiment of the present disclosure to provide a non-viral strategy based on the use of the system of nanoparticles denominated neurotensin polyplex for targeted delivery of the BDNF transgene under the control of hDAT promoter to the dopaminergic neurons of the substantia nigra in experimental conditions (e.g., non-human animals, preferably rats) with the consequent reduction of early hemiparkinsonism caused by a significant damage of the dopaminergic neurons with 6-OHDA, a damage fully manifested by severe and evident motor impairment, comprising the use of the non-viral method for such experimental purposes.

It is set as embodiment of the present disclosure to provide a non-viral strategy based on the use of the system of nanoparticles denominated neurotensin polyplex for the targeting delivery of BDNF transgene under the control of hDAT promoter to the dopaminergic neurons of the substantia nigra in experimental conditions (e.g., non-human animals) with the consequent reduction of so caused parkinsonism by the selective destruction of the nigrostriatial dopaminergic neurons by the systemic administration of neurotoxin 1-methy-4-phenyl-1,2,3,6-tetrahydropyridine (MPTP, used as idiopathic PD model) (Luquin et al., 1991).

Embodiments of the present invention are the pharmaceutical compositions, their production for use in the treatment of PD, or for application e.g., in experimental models for studies of PD, wherein said pharmaceutical compositions are characterized by comprising as active component the macromolecule that functions as carrier of the neurotensin denominated NTS-polyplex, constituted in turn by the neurotensin ligand (NTS) and the fusogenic peptide (PF), which can be the derivative H2A hemagglutinin of influenza virus (modified) with the sequence GLFEAIAEFIEGGWEGLIEGCAKKK, binded by the cross-linking LC-SPDP ((Succinimidyl 6 - (3 - [2-pyridyldithio]-propionamyde) hexanoate)) to poly-L-lysine (PLL) that binds a plasmid encoding the gene for BDNF, which may be the plasmid phDAT-BDNF-flag (10.511 Kbp), in which the gene encoding for BDNF is under control of hDAT promoter (dopamine transporter). The NTS-polyplex designed to perform the present invention is conformed by the inclusion of a caryophillic peptide (PK) as nuclear localization signal; this can be the one of the major capsid protein of simian virus 40 (SV40) with the aminoacid sequence MAPTKRKGSCPGAAPNKPK, which is electrostatically attached to the plasmid (Martinez-Fong et al., 2012).

Within the scope of the present disclosure are the methods to study in experimental models *in vitro* or *in vivo* the regulatory mechanisms of BDNF neurotrophin expression, or cellular events associated with neurotrophism by the transfection of neurons possessing the internalization receptor for Neurotensin with the transgene encoding for BDNF using the NTS-polyplex of the present invention as carrier; the administration by parenteral injection, or in the cerebrospinal fluid, or *in situ* at the basal ganglia level of the brain; the application consistent with adequate assessment methods of the expected responses by the BDNF transgene expression. The responses associated to neurotrophism by the effect of BDNF may include but are not limited to the following: cellular and molecular mechanisms of axonal regeneration, neuronal metabolism, neuronal bioenergetics, neurotransmitter activity and signaling events of apoptosis, or the effect of polymorphisms of these proteins.

Within the embodiments of the present disclosure is the ability to design experimental models both *in vitro* and *in vivo* for PD, while based on the used of NTS-polyplex just as it is implicit in the present disclosure.

The following examples should not be seen as to limit the scope of the present disclosure; they are only intended to illustrate its performance and are a part of the required experimental support in the technological field of the invention.

**Example 1. Construction of plasmids.** The plasmid phDAT-EGFP-N1 (10.45 Kbp), encoding the green fluorescent protein (GFP) under control of the promoter hDAT (human dopamine transporter), was obtained from the cloning of the 5' end of the regulatory region of 6250 bp of hDAT in the Eco47III / BgIII sites of pCMV-EGFP1-N1 (Clontech, Palo Alto, CA, USA) (Bannon et al., 2001; Sacchetti et al., 1999). Figure 2 shows the simplified restriction map of phDAT-EGFP-N1.
The plasmid phDAT-BDNF-flag (10.511 Kbp), encoding the BDNF-flag gene under control of the promoter hDAT, was used as neurotrophic gene in the present disclosure. The plasmid phDAT-BDNF-flag was obtained from cloning BDNF-flag of 868 bp into the Notl / Sail sites of phDAT-EGFP-N1. In a first step, the removed 847 bp restriction fragment from pAD1-BDNF-flag plasmid using Hind III enzyme and Notl was subcloned into the pBluescript SK (+) plasmid, thus generating the plasmid pBluescript SK(+)-BDNF-flag. In this plasmid, we sequenced the fragment of 847 bp encoding for BDNF, the flag label, and the flanking restriction sites. Subsequently, the transgene BDNF-flag of 868 bp was removed with the restriction enzymes Xhol / Notl from the pBluescript SK(+)BDNF-flag plasmid and was subcloned into the Sail / Notl sites of the phDAT-EGFP-N1 plasmid, replacing the fragment EGFP-N1 (762 bp) and finally generating the working plasmid phDAT-BDNF-flag (10.511 Kbp). Figure 2 shows the simplified restriction map of phDAT-BDNF-flag.

**Example 2. Synthesis of NTS-polyplex.** We make here a brief description of the synthesis of NTS-polyplex, because the detailed procedure can be found in previous publications from our laboratory (Arango-Rodriguez et al., 2006; Martinez-Fong and Navarro-Quiroga, 2000; Navarro-Quiroga et al., 2002). In a first step, the NTS (Sigma; Saint Louis, MO, USA) and modified PF-hemagglutinin-HA2 of influenza virus (GLFEAIAEFIEGGWEGLIEGCAKKK; >90% purity; SynPep; Dublin, CA, USA) were conjugated with poly-L-lysine (PLL; average molecular mass 48 kD) using LC-SPDP crosslinker. The SPDP derivatives and the NTS-SPDP-(PF-SPDP)-PLL conjugate were purified by size exclusion chromatography; the latter is known as "NTS carrier." The NTS carrier was dialyzed into a phosphate buffer solution (PBS pH 7.4) and concentrated to a final volume of 1 mL. Subsequently, this concentrate was sterilized by membrane filtration of 0.2 µm and preserved at -70°C until use.
In a second step, we performed the assembly of NTS-polyplex comprising the electrostatic binding of DNA plasmid with the caryophillic peptide (MAPTKRKGSCPCAAPNKPK; 90% purity; Synpep Corp., Dublin, CA, USA) and subsequent electrostatic binding of these with the NTS-carrier, following the procedure described previously (Arango-Rodriguez et al., 2006; Navarro-Quiroga et al., 2002). We determined the optimal molar ratio of the components of NTS-polyplex using electrophoretic retardation and retention assays, which allow anticipating the applicable concentration of DNA, PK, and the NTS-carrier so resulting in an efficient NTS-polyplex (figure 3). The optimal concentration of DNA and PK was calculated based on the electrophoresis moving pattern by agarose gel of the DNA-PK complexes formed at a constant concentration of DNA (6 nM) with increasing concentrations of PK (retardation assay) (Arango-Rodriguez et al., 2006; Martinez-Fong et al., 1999; Martinez-Fong and Navarro-Quiroga, 2000; Navarro-Quiroga et al., 2002). The optimal molar ratio was that where the first evident delay in the migration of the band with respect to DNA alone (Martinez-Fong and Navarro-Quiroga, 2000; Martinez-Fong et al., 2012; Navarro-Quiroga et al., 2002). In this example, the first delay of the DNA-PK complexes showed at a concentration of 5 µM PK. To the DNA-PK complex formed at the optimal molar ratio was added 1% inactivated fetal bovine serum to produce a higher condensation before adding NTS carrier (Arango-Rodriguez et al., 2006). Finally, we obtained the optimal relation of DNA-PK complex:NTS carrier, using retardation assays (Martinez-Fong and Navarro-Quiroga, 2000; Martinez-Fong et al., 2012; Navarro-Quiroga et al., 2002). For this purpose, a constant ratio of DNA-PK complex (6 nm : 5 µM) was incubated with increasing concentrations of the carrier and after the electrophoresis moving pattern by agarose gel at 0.8%, we chose the NTS carrier relation causing the last visible retention of the DNA band in the source well, which for this example were 144 nM of NTS carrier (figure 3). In summary, the optimal molar ratios of the NTS-polyplex components were 6 nM of DNA plasmid (phDAT-EGFP or phDAT-BDNF-flag), 5 µM PK, 144 nm NTS carrier (ratio 1 : 24), and 1% inactivated fetal bovine serum (figure 3).
In the transfection examples with phDAT-EGFP and phDAT-BDNF-flag for the present disclosure, a solution 5X of NTS-polyplex at optimal molar ratio was used; that is 30 nM of plasmid, 25 µM PK, 720 nM NTS carrier, and 5% of inactivated bovine fetal serum.
At the optimal molar ratio, the NTS-polyplex nanoparticles resulting from the compaction of DNA plasmid by poly-L-lysine have the proper molar concentration of NTS, PF, and PK for efficiently and selectively performing the gene transfer as shown in figure 1.

**Example 3. Lesion and transfection method.** We used male rats of Wistar strain, weighing an average of 210-230 grams and subjected them to stereotactic surgery to generate the lesion with 6-OHDA or false lesion (FLN) and transfected with the NTS-polyplexes, or injected with DMEM (false transfection). Prior to stereotactic surgery, the animals were anesthetized by intraperitoneal administration of ketamine (70 mg/Kg)-xylazine (6 mg/Kg). Under deep anesthesia, the animals were placed in the stereotaxic apparatus. After cranial trepanation with a dental drill, the animals received a single injection of 3 µL of the 6-OHDA neurotoxin solution (6.7 µg free base/ µL phosphate buffer 0.1 M (PBS) containing 0.2% ascorbic acid as antioxidant). 6-OHDA was prepared at the time of application and preserved at 4°C in the dark to avoid degradation. Control animals with the false lesion (FLN) received only 3 µL of PBS with 0.2% ascorbic acid. In both cases, the neurotoxic and control solutions were administered using a dental needle at a flow of 0.25 µL/min. After each injection, diffusion was allowed during 5 minutes and right afterward the dental needle was slowly removed. The coordinates used for the lesion or false lesion in the left striatum were: AP, 0 mm with reference to Bregma; ML + 4 mm, DV, 5.2 mm from the dura (figure 3). These coordinates correspond to the front and dorsal striatum of the anterior commissure using as a reference the atlas of Paxinos and Watson. After surgery, the animals were placed in their cages where they remained for the next 7 days for recovery. The animals received food and water *ad libitum* in a normal light-dark cycle of 12:12.
One week after the lesion, transfection was performed in the CNS ipsilateral to the lesion (figure 4). The stereotaxic coordinates were: AP, -5.4 mm from Bregma; ML, + 1.5 mm, DV -6.8 mm from the dura. Each animal received a single intranigral dose of 3 µL of NTS-polyplex or DMEM (which we call false transfection) at a flow of 0.25 µL/min. The total amount of plasmid injected in 3 µL was 555 ng for phDAT-BDNF-flag and 631 ng for phDAT-EGFP.

**Example 4. Evaluation of the presence of NTSR1 and transgene expression by RT-PCR.** The technique of reverse transcription and polymerase chain reaction (RT-PCR) was used to demonstrate the presence of mRNA for NTSR1, GFP, and BDNF-flag in the surviving dopaminergic neurons of the CNS one week after the lesion for the case of NTSR1 and 3 weeks after the lesion for the transgene expression. The expression of the constitutive gene for actin was used as internal control. The RNA was extracted using Trizol following the procedure previously described (Orozco-Barrios et al., 2009). Total RNA was treated with DNase free from RNase and transcribed with reverse transcriptase enzyme SuperScript II (200 U) using 1 µg oligo dT (Invitrogen Corporation, Carlsbad, CA, USA). cDNA products were amplified in a thermocycler (Gene Amp PCR System 9700; Applied Biosystems, Foster City, CA, USA) using 1 nmol of first sense and antisense and 1.5 U of DNA Taq polymerase (Invitrogen Life Technologies, San Diego, CA, USA) at a final volume of 25 µL. To amplify a fragment of 537 bp of NTSR1, the first sense was 5'-CGTAAAGACCTCTATGCCAA-3', and the first antisense was 5'-ACTCCTGCTTGCTGATCCAC-3'. To amplify a fragment of 608 bp of the GFP the first sense was 5'-CTGGTCGAGCTGGACGGCGAC-3', and the first antisense was 5'-AGAGTGATCCCGGCGGCGGTC-3'. To amplify a fragment of 163 bp of BDN-flag the first sense was 5'-GCAATGCCGAACTACCCAATC-3', and the first antisense was 5'-CTTGTCATCGTCGTCCTTGTAGTC-3'. To amplify a fragment of 349 bp of actin, the first sense was 5'-CGTAAAGACCTCTATGCCAA-3', and the first antisense was 5'-ACTCCTGCTTGCTGATCCAC-3'. After initial denaturation at 94°C for 5 min, amplification was performed with 35 cycles for GFP, BDNF-flag and actin, and 40 cycles for NTSR1 as follows: denaturation, 94°C during 1 min; tempering, 60°C for NTSR1 and actin; 63°C for BDNF-flag and 66°C for GFP; extension, 72°C during 30 s for BDNF-flag and actin; 36 s for GFP and 45 s for NTSR1. The PCR products (amplicons) for NTSR1, GFP, and actin were analyzed by electrophoresis on 2% agarose gel, and 5% polyacrylamide for BDNF-flag. Bands were strained with ethidium bromide and photographed with a Kodak camera DC290.
The photograph of the agarose gel samples from healthy and lesioned CNS shows the electrophoretic band of NTSR1 amplicon (figure 5A). The NSTR1 amplicon band is also observed in samples from N1E-115 cells (figure 5A, lane 1), which are the positive control of the expression of this receptor (Martinez-Fong et al., 1999). These results demonstrate that the dopaminergic neurons originally expressing NTSR1 retain this receptor 8 days after being subjected to the insult induced by 6-OHDA striatal injection. At this time, the hemiparkinsonian rats were transfected in the CNS ipsilateral to the lesion with plasmid phDAT-EGF or phDAT-BDNF-flag using NTS-polyplex.
Two weeks after transfection (three weeks after the lesion), the mRNA expression for GFP and BDNF-flag was evaluated in surviving dopaminergic neurons. Panel A of figure 6 shows the 608 bp band separated by electrophoresis corresponding to the GFP transcript only amplified in the transfected side of CNS (lane 3). Similarly, panel A of figure 7 shows the 163 bp band corresponding to the BDNF-flag transcript only amplified in the transfected side of the CNS (lane 3). Lane 1 of figures 6 and 7 show the amplifying band of plasmids phDAT-EGFP and phDAT-BDNF-flag, respectively, used as positive control of PCR technique. In lanes 4 and 5, samples of the striata were run where the amplicon band is not observed because they were not transfected (negative control). These results suggest that dopaminergic neurons surviving the lesion with 6-OHDA were able to express GFP and BDNF-flag two weeks after the transfection with the NTS-polyplex.

**Example 5. Evaluation of the presence of NTSR1 and the transgenic expression by immunofluorescence.** Double immunofluorescence was used to demonstrate the presence of NTSR1 and the expression of GFP or BDNF-flag in the surviving dopaminergic neurons of the CNS one week after the lesion for the case of NTSR1 and three weeks after the lesion for the transgenic expression. Briefly, a series of the coronal sections of 30 µm of the rat's midbrain of each experimental condition was processed as follows. Nonspecific binding sites of the antibody were blocked with a PBS solution with 5% PBS horse serum for 2 hours at room temperature. After washing with PBS, the tissues were permeabilized with 0.3% PBS Triton X-100. After being permeabilized, sections were incubated for 16 hours at 4°C with the pair of primary antibodies, one of them was a mouse monoclonal and the other was goat or rabbit polyclonal. After washing with PBS, sections were incubated with secondary antibodies labeled with suitable fluorescein or rhodamine (dilution 1:400). Negative controls were obtained by omitting the primary antibody. Finally, the fluorescence in the cells was recorded by a TCS-SPE two photon laser-scanning microscope (Leica Microsystem, Wetzlar Germany) using a 60 X oil immersion objective at excitation/emission wavelength of 488-522 nm (green channel) and 568-635 nm (red channel). Consecutive optical sections were obtained from twenty to forty at intervals in the range of 0.3 µm at z series. The resulting images were projected onto a bidimensional plane and were superimposed on the screen of a monitor using the green color for fluorescein and red for rhodamine or Texas red.
One week after the 6-OHDA lesion, the presence of NSTR1 in the dopaminergic neurons was evaluated by double immunofluorescence. The primary antibodies were a monoclonal antibody against TH made in mouse (1:400 dilution), and a polyclonal antibody against NTSR1 made in goat (dilution 1:400), both obtained from Sigma-Aldrich, St. Louis, MO. The secondary antibodies were IgG anti-mouse labeled with fluorescein made in donkey and IgG anti-goat labeled with Texas red made in rabbit (Jackson ImmunoResearch Laboratories Inc.; West Grove, PA, USA). Confocal microscopy analysis shows that one week after the 6-OHDA lesion, the remaining nigral neurons are immunoreactive to TH (green channel) and NTSR1 (red channel), as the CNS cells of the control side (figure 5B). These results show that the dopaminergic neurons originally expressing NTSR1 retain this receptor 8 days after being subjected to the insult induced by striatal injection of 6-OHDA. At this time, the hemiparkinsonian rats were transfected in the CNS ipsilateral to the lesion with plasmid phDAT-EGF or BDNF-flag using NTS-polyplex.
Two weeks after the transfection (three weeks after the lesion), the expression of the transgenic proteins in the surviving dopaminergic neurons was evaluated by double immunofluorescence. The primary antibodies to show the expression of GFP or BDN-flag in the dopaminergic neurons were a polyclonal antibody against TH made in rabbit (1:400 dilution; (1:300 dilution; Chemicon; Temecula, CA) and a monoclonal antibody against GFP or against lag made in mouse (1:400; Sigma-Aldrich; St. Louis, MO). The secondary antibodies were anti-rabbit made in goat labeled with fluorescein (1:400 dilution; Jackson ImmunoResearch Laboratories Inc.; West Grove, PA, USA) and anti-mouse made in donkey labeled with rhodamine. The analysis of the images taken with confocal microscopy revealed that, two weeks after the transfection with phDAT-EGFP-N1 plasmid, the GFP immunoreactivity (red channel) was located in the neurons immunoreactive to TH (green channel) of the CNS transfected and lesioned (experimental side) and was absent in the untransfected CNS (figure 6B). Similarly, the confocal analysis showed that the immunoreactivity of Flag (red channel) was located only in the few TH+ neurons (green channel) of the lesioned CNS transfected with phDAT-BDNF-flag (figure 7B). These results suggest that dopaminergic neurons surviving the 6-OHDA lesion were able to express GFP and BDNF-flag two weeks after transfection with NTS-polyplex.

**Example 6. Morphological evaluation of the neurotrophic effect.** Two weeks after transfection into hemiparkinsonian rats, the effect of the BDNF-flag expression on the number of dopaminergic neurons and neuritic branches in the CNS and on the striatal innervation was evaluated using immunohistochemistry for TH. Briefly, following behavioral assessment, the animals were transcardially perfused with 30 mL of 0.1 M PBS followed by 250 mL of 4% paraformaldehyde in PBS. The brains were carefully removed from the skulls and cryoprotected in a solution of 30% sucrose at 4°C for two days. Then, using a freeze sliding microtome, coronal sections of 30 µm thick were made at CNS striatum level. For both areas, we collected all consecutive sections that were distributed in five series to obtain comparative levels in the anterior-posterior axis of the CNS and striatum. Only one of the series, randomly selected, was processed with immunohistochemistry against TH, a marker of dopaminergic neurons in the midbrain. After washing the sections with PBS, they were incubated with a PBS solution with 1% hydrogen peroxide and 2% horse serum for 2 hours at room temperature. Subsequently, the sections were permeabilized with 0.3% PBS Triton X-100 (Sigma-Aldrich; St. Louis, MO) and incubated with the primary anti-TH monoclonal antibody made in mouse (Sigma-Aldrich; St. Louis, MO) (1:1000 dilution) for 16 hours at 4°C. After washing with 0.02% PBS Triton X-100, the sections were incubated with a biotinylated secondary antibody anti-mouse made in horse (Vector Laboratories, Burlingame CA) at 1:200 dilution for 2 hours at room temperature. The visualization of immunoreactive sites was performed using a peroxidase enzyme reaction using diaminobenzidine as substrate, according to the manufacturer's instructions (VectaStain Elite ABC, Vector Labs). Following the immunohistochemistry process the preparations were washed with PBS and mounted on gelatinized slides, and covered with the polymer Entellan (Merck KGaA). The recording of the information obtained from previous immunohistochemistry was performed obtaining digitalized images with a Leica DMIRE2 DC300F microscope (Nussloch, Germany). Bright field images of TH immunoreactivity in the CNS and the striatal nucleus in the different experimental conditions were obtained with a Leica DMIRE2 microscope and digitalized with a Leica DC300F camera (Nussloch, Germany).
Three weeks after instratial injection of 6-OHDA we observed a dramatic decrease in the immunoreactivity to TH in the CNS and striatum compared to the unlesioned sides (figures 8 and 9). Unlike control hemiparkinsonian animals (non-transfected, injected with DMEM, or transfected with GFP), the animals transfected with phDAT-BDNF-flag developed a robust arborization of TH+ fibers in the CNS transfected, but its density was lower than in normal CNS (figure 8). Similarly, the previously damaged striated, ipsilateral to the CNS transfected with phDAT-BDNF-flag plasmid showed a significant recovery of TH-immunoreactivity compared with the striatal nucleus of the negative control animals (figure 9). These results suggest that the expression of BDNF-flag induced the arborization of dopaminergic neurons in the CNS and the recovery of dopaminergic innervation of the striatum.

**Example 7. Cell count.** To perform the counts of the dopaminergic neurons of the CNS of each experimental group, the immunohistochemical preparations were counterstained with cresyl violet and covered with the polymer Entellan (Merck KGaA). We obtained bright-field images using the objective 20X of Leica DMIRE1 DC300F microscope (Nussloch, Germany). We took an average of 40 micrographs of each brain using a Leica DC300F camera (Nussloch, Germany). A total of 15 animals (3 for each experimental group) were considered for counting TH immunoreactive neurons. Only those neurons with intense immunoreactivity were considered in the count. To include in the cell count mainly those neurons located in the CNS, we considered the interpeduncular space as medial dividing line between the left and right CNS and the ventral tegmental area (Oades and Halliday, 1987).
When performing the counts of TH+ neurons in each condition, it was found that all the experimental conditions showed a significant decrease (P < 0.001) of 80% vs. the contralateral population of the CNS in the same brains (figure 8). These results demonstrate that the short-term effect of BDNF-flag is exerted on the neuritic tree and not only on the neurogenesis.

**Example 8. Dopamine quantification.** To determine whether the increase in TH immunoreactivity was correlated with increased levels of the neurotransmitter, we measured the dopamine content for reverse HPLC and electrochemical detection in homogeneous and striated supernatants of the CNS. Briefly, brains were removed and placed in a cold ice plate to dissect the CNS and striatum that were immediately frozen on dry ice. The CNS and striatum tissue samples were homogenized in 50 µL and 100 µL of 0.1 M perchloric acid, respectively, and centrifuged at 20,000 g during 2 min (Airfuge, Beckman, Palo Alto, CA, USA). After filtration, 5 µL of supernatants were injected into a reverse phase HPLC apparatus using a Rheodyne injector. The analytic column was a Unijet C-18 (3 µm, 100 mm x 3.2 mm; Bioanalytical Systems; West Lafayette, Indiana, USA), through which the mobile phase was pumped (monobasic sodium phosphate, 25 mM; sodium citrate, 50 mM; EDTA, 0.03 mM; diethylamine HCl, 10 mM; octylshulphonic acid, 2.2 mM; 30 mL methanol, and 22 mL dimethylacetamide, pH 3.0) at a flow of 0.5 mL/min. Dopamine was measured by electrochemical detection using a LC-4 C amperometer detector (Bioanalytical Systems; West Lafayette, Indiana, USA). The glassy carbon electrode was set at a potential of + 0.75 volts over the reference electrode (3 M KCl). We integrated the peak areas of dopamine to calculate the concentrations in the sample using the software ChormGraph Report (Bioanalytical Systems; West Lafayette, Indiana, USA). The dopamine levels were expressed as picograms per microgram of protein.
The HPLC results obtained show that the dopamine level in the CNS in all experimental conditions showed a significant reduction of 70% vs. their intact counterparts, three weeks after the ipsilateral striatum lesion (figure 10B). Similarly, the striatal dopamine decreased in approximately 70% vs. the intact contralateral striata, two weeks after transfection with GFP, the false transfection with DMEM, or the non-transfection as shown in figure 10B. In contrast, the dopamine level found in the striata of hemiparkinsonian animals transfected with BDNF-flag recovered 75% of the total dopamine contents determined in their intact counterparts (figure 10A). This result is consistent with the recovery of striatal innervation of TH immunoreactivity and suggests that the recovery of the neurotransmitters in the target innervation nucleus of the dopaminergic neurons of the CNS could lead to the recovery of the motor function affected in Hemiparkinsonism.

**Example 9. Improved spontaneous motor behavior.** Spontaneous Motor Behavior in Open Field is an indicator of movement that each animal exhibits in basal conditions, and therefore is susceptible to bleaching by factors that stimulate, inhibit, or alter normal motor characteristics. Such is the case of some drugs, stress, or lesion in brain areas related to movement, as induced by 6-OHDA.

To assess the effect of BDNF-flag transfection in hemiparkinsonian rats, the animals were subjected to the spontaneous motility test in the open field for 30 minutes. With the aim to avoid interference with the pharmacological rotation tests, this spontaneous motility test was conducted at the beginning of each series of behavioral assessments. To avoid the possible effect of stress on motility, the animals were habituated to the experimental room 24 hours before the experiment. Motor activity was evaluated in a new environment by placing individual animals in a square wooden box (60 cm X 60 cm X 50 cm high per wall) painted in black and placed in the center of the evaluation room (Zamudio et al., 2005). The total testing time was 30 minutes divided into intervals of 3 minutes. After each experiment, the box was cleaned to avoid olfactory signal interference between consecutive animals. Motor activity was video-recorded and analyzed on a Videomex-V computer (Columbus, OH, USA). The motion parameters analyzed were: the distance traveled, ambulatory movement time, rest time, and the movement time considered stereotyped as scratching, short stroke (and repetitive) of the head, and body, and facial grooming. To avoid the possible effect of learning, this test was performed only once for each animal as the beginning of the behavioral assessment after each treatment.
Three weeks after 6-OHDA injection, the hemiparkinsonian rats remained at rest for longer time and (figure 11C) exhibited lower motor performance vs. the rats with a false lesion in terms of distance traveled, time moving and stereotypic movements (figures 11A, 11B, and 11D). Two weeks after transfection with BDNF-flag, the hemiparkinsonian animals significantly increased the total distance traveled vs. the groups receiving no transfection or transfected with a reporter gene (figure 11A). The values in ambulatory and stereotyped movement of hemiparkinsonian animals treated with BDNF-flag were higher than those obtained in untransfected hemiparkinsonian groups or transfected with GFP, and showed no significant differences from the control group (figures 11B and 11D). Consistently, hemiparkinsonian animals treated with BDNF-flag remained less time at rest than the untransfected (NT) and the false-transfected animals (DMEM) (figure 11D). In summary, animals treated with BDNF-flag showed no statistically significant differences in any of the motor parameters evaluated in the spontaneous motility test in open field vs. the false lesion group (FLN). We conclude then that the improved behavior could be owed to the recovery of dopaminergic innervation and dopamine levels in the striatum induced by the expression of BDNF-flag after being transfected with the NTS-polyplex.

**Example 10. Improvement of behavior changes induced by dopaminergic drugs.** Behavioral tests where drugs are used with an effect on the dopaminergic system allow the detection of motor disturbances, which otherwise would be imperceptible. This is the case of the rotational behavior induced by amphetamine, a competitor of catecholamine vesiculation and the apomorphine, a non-selective dopaminergic agonist, both of which allow to clearly demonstrating the motor impairment produced by Hemiparkinsonism in rats. Both rotational behaviors induced by these drugs have been widely used to assess the imbalance of dopamine released in the striatum (amphetamine) and the development of hypersensitivity to dopamine receptors in the denervated striatum (apomorphine), in murine models of Hemiparkinsonism.
To demonstrate the recovery of motor function induced by the expression BDNF-flag in hemiparkinsonian rats we used both drugs independently, according to the following protocol: the animals on the different treatments received an intraperitoneal injection of 8 mg/Kg/mL of methamphetamine (Sigma-Aldrich; MO); after which they were placed within an automated gyroscope, loosely gripped to a cable through a harness, to allow free movement. The asymmetry in both dopaminergic systems caused by the lower amount of dopamine in the lesioned side was assessed by counting the full rotations in ipsilateral direction to the lesion induced by amphetamine for 90 minutes. Amphetamine-induced rotational behavior was assessed 8 days after the lesion to reveal the established dopaminergic neurodegeneration and select the groups subjected to transfection. The rotational behavior equal or greater than 1000 ipsilateral turns in 90 min was established as a selection criterion to assign the animals to the groups of transfection with BDNF-flag or GFP, DMEM injection (false transfection), or no transfection. Two weeks after transfection, we assessed again the rotational behavior stimulated by amphetamine in the groups of the animals, which were previously selected and assigned to said treatments. The same administration protocol was used for apomorphine, whose role is to demonstrate and quantify the asymmetry in both dopaminergic systems caused by hypersensitivity of postsynaptic dopaminergic receptors in the denervated side. To this we administrated subcutaneously 0.5 mg/Kg/mL of apomorphine (Sigma-Aldrich; St. Louis, MO, USA); after which the animals were placed individually in the gyro meters. This behavior was evaluated 9 days after the lesion and two weeks (+ 1 day) after the transfection with BDNF-flag, GFP, false transfection with DMEM, or absence of transfection.
Three weeks after the lesion, the groups of hemiparkinsonian groups with false transfection applying only the vehicle of NTS-polyplex (DMEM) or with no transfection (NT) exhibited values of ipsilateral rotational behavior induced by amphetamine similar to those shown on the week after the lesion, indicating the stability of the dopaminergic neurodegeneration at that time of evaluation (figure 12). Similarly, the group of hemiparkinsonian rats transfected with GFP non-neurotrophic gene did not show a decrease in the ipsilateral rotational behavior two weeks after the transfection. By contrast, animals transfected with the neurotrophic gene BDNF-flag showed a significant reduction of 72% in the number of amphetamine-induced rotations respect to the number of rotations in the precondition of transfection (figure 12D). The decrease in rotational behavior induced by amphetamine in hemiparkinsonian animals transfected with BDNF-flag reflects the recovery of dopamine on the lesioned side, as shown by HPLC (figure 10A), and therefore is a clear evidence of the decreased Hemiparkinsonism.
In these experimental conditions, the average number of contralateral rotations induced by apomorphine one week after the 6-OHDA lesion in hemiparkinsonian animals was 95 ± 25 rotations in 90 min. While two weeks after the intranigral transfection, the average of contralateral rotations in the control hemiparkinsonian animals was 255 ± 5 rotations in 90 min, which represents an increase of 168% vs. the number of rotations performed before the transfection (figure 13, A-C). In contrast, the group of hemiparkinsonian rats transfected with BDNF-flag developed 161 ± 32 ipsilateral rotations in 90 min, which were 63% lower (P < 0.05) than the rotations in control hemiparkinsonian groups (figure 13D). The decrease in the rotational behavior induced by apomorphine in the hemiparkinsonian animals transfected with BDNF-flag reflects the decrease of the hypersensibility of postsynaptic dopaminergic receptors on the lesioned side owed to a recovery of dopamine and constitutes another evidence of the decrease of Hemiparkinsonism.
The results of the rotational behavior induced by these dopaminergic drugs are consistent with the improvement in spontaneous behavior in the group of animals transfected with BDNF-flag and constitute an evidence of the therapeutic effect of BDNF gene transfer through the NTS-polyplex on Parkinsonism induced by 6-OHDA. The NTS-polyplex was able to transfect the surviving dopaminergic neurons surviving to a severe lesion of 6-OHDA because they retained the functional expression of NTSR1. Several clinical studies have shown the presence of mRNA and NTSR1 protein in the nigral dopaminergic neurons of patients with PD (Sadoul et al., 1984; Uhl et al., 1984; Yamada and Richelson, 1995). These findings support the possibility of using the NTS-polyplex to transfect the BDNF gene into the surviving dopaminergic neurons in early to intermediate stages of PD as a neurotrophic therapy approach. Since the dopaminergic neurons of patients with PD also retain the presence of TrkB, BDNF expressed by the transfection with NTS-polyplex is capable of activating these receptors to produce the morphological, biochemical, and behavioral improvements showed in the present disclosure in parkinsonian patients.

### References.

1. Airaksinen, M.S., et.al. 2002. Nat Rev Neurosci. 3, 383-94.
2. Alderson, R.F., et.al. 1990. Neuron. 5, 297-306.
3. Alonso-Vanegas, M.A., et.al. 1999. J Comp Neurol. 413, 449-62.
4. Alvarez-Maya, I., et.al. 2001. Mol Med. 7, 186-92.
5. Arango-Rodriguez, M.L., et.al. 2006. Biochim Biophys Acta. 1760, 1009-20.
6. Baker, S.A., et.al. 2005. Brain Res. 1039, 177-88.
7. Bamji, S.X., et.al. 1998. J Cell Biol. 140, 911-23.
8. Bannon, M.J., et.al. 1997. Neurology. 48, 969-77.
9. Bannon, M.J., 2005. Toxicol Appl Pharmacol. 204, 355-60.
10. Baquet, Z.C., et.al. 2005. J. Neurosci. 25, 6251-9.
11. Barde, Y.A., et.al. 1982. EMBO J. 1, 549-53.
12. Bartus, R.T., et.al. 2011. Mov Disord. 26, 27-36.
13. Benisty, S., et.al. 1998. Neuroscience. 86, 813-26.
14. Bennet, M.R., et.al. 2002. Auton Neurosci. 95, 1-23.
15. Bosse, K.E., et.al. 2012. J. Neurochem. 120, 385-95.
16. Brooks, A.R., et.al. 2004. J. Gene Med. 6, 395-404.
17. Bryans, M., et.al. 1992. FEBS Lett. 309, 97-102.
18. Canudas, A.M., et.al. 2005. Brain Res Mol Brain Res. 134, 147-54.
19. Casaccia-Bonnefil, P., et.al. 1999. Microsc Res Tech. 45, 217-24.
20. Castel, M.N., et.al. 1994. Biochem Pharmacol. 47, 53-62.
21. Costall, B., et.al. 1976. Brain Res. 118, 87-113.
22. Diederich, N.J., et.al. 2003. Arch Neurol. 60, 529-33.
23. Frim, D.M., et.al. 1994. Proc Natl Acad Sci U S A. 91, 5104-8.
24. Garcia Navia, J.T., et.al. 2008. J Neurosci Res. 86, 2016-27.
25. Georgievska, B., et.al. 2002. Exp Neurol. 177, 461-74.
26. Ghosh, A., et.al. 1994. Science. 263, 1618-23.
27. Gonzalez-Barrios, J.A., et.al. 2006. Mol Ther. 14, 857-65.
28. Goulet, M., et.al. 1999. Synapse. 32, 153-64.
29. Gray, K., et.al. 2008. FEBS Lett. 582, 907-10.
30. Hagg, T. 1998. Exp Neurol. 149, 183-92.
31. Hallbook, F., et.al. 2006. Brain Behav Evol. 68, 133-44.
32. Herzog, C.D., et.al. 2009. Neurosurgery. 64, 602-12; discussion 612-3.
33. Howells, D.W., et.al. 2000. Exp Neurol. 166, 127-35.
34. Huang, E.J., et.al. 2001. Annu Rev Neurosci. 24, 677-736.
35. Hyman, C., et.al. 1991. Nature. 350, 230-2.
36. Johnson, J.E., et.al. 1986. J Neurosci. 6, 3031-8.
37. Kirik, D., et.al. 1998. Exp Neurol. 152, 259-77.
38. Klein, R.L., et.al. 1999. Brain Res. 847, 314-20.
39. Koshimizu, H., et.al. 2010. Neurosci Lett. 473, 229-32.
40. Lapchak, P.A., et.al. 1993. Neuroscience. 53, 639-50.
41. Levivier, M., et.al. 1995. J Neurosci. 15, 7810-20.
42. Lindholm, D., et.al. 1996. Eur J Neurosci. 8, 1452-60.
43. Lowenstein, D.H., et.al. 1996. J Neurosci. 16, 1759-69.
44. Luquin, M.R., et.al. 1991. Neurologia. 6, 287-94.
45. Madara, J.C., et.al. 2008. J Neurophysiol. 100, 3175-84.
46. Maisonpierre, P.C., et.al. 1991. Genomics. 10, 558-68.
47. Marks, W.J., Jr., et.al. 2010. Lancet Neurol. 9, 1164-72.
48. Martinez-Fong, D., et.al. 1999. Brain Res Mol Brain Res. 69, 249-62.
49. Martinez-Fong, D., et.al. 2000. Brain Res Brain Res Protoc. 6, 13-24.
50. Martinez-Fong, D., et.al. 2012. Nanomedicine 8 (7), 1052-1069.
51. Masuo, Y., et.al. 1990. Brain Res. 510, 203-10.
52. Mowla, S.J., et.al. 2001. J Biol Chem. 276, 12660-6.
53. Murer, M.G., et.al. 2001. Prog Neurobiol. 63, 71-124.
54. Navarro-Quiroga, I., et.al. 2002. Brain Res Mol Brain Res. 105, 86-97.
55. Nishio, T., et.al. 1998. Neuroreport. 9, 2847-51.
56. Nouel, D., et.al. 1997. J Neurosci. 17, 1795-803.
57. Numan, S., et.al. 1999. J Comp Neurol. 403, 295-308.
58. Numan, S., et.al. 2005. J Mol Neurosci. 27, 245-60.
59. Oades, R.D., et.al. 1987. Brain Res. 434, 117-65.
60. Oo, T.F., et.al. 2009. Mol Cell Neurosci. 41, 440-7.
61. Orozco-Barrios, C.E., et.al. 2009. PLoS One. 4, e8268.
62. Ostergaard, K., et.al. 1996. Exp Neurol. 142, 340-50.
63. Palacios, J.M., et.al. 1981. Nature. 294, 587-9.
64. Parain, K., et.al. 1999. Neuroreport. 10, 557-61.
65. Patapoutian, A., et.al. 2001. Curr Opin Neurobiol. 11, 272-80.
66. Porritt, M.J., et.al. 2005. Exp Neurol. 192, 226-34.
67. Ramaswamy, S., et.al. 2009. Neurobiol Dis. 34, 40-50.
68. Ramirez-Jirano, L.J., et.al. 2007. Rev Neurol. 44, 15-7.
69. Rubio-Zapata, H.A., et.al. 2009. Cancer Gene Ther. 16, 573-84.
70. Sacchetti, P., et.al. 1999. Brain Res Mol Brain Res. 74, 167-74.
71. Sadoul, J.L., et.al. 1984. Biochem Biophys Res Commun. 125, 395-404.
72. Sajadi, A., et.al. 2005. J Neurochem. 93, 1482-6.
73. Sauer, H., et.al. 1993. Brain Res. 626, 37-44.
74. Sauer, H., et.al. 1994. Neuroscience. 59, 401-15.
75. Seidah, N.G., et.al. 1996. FEBS Lett. 379, 247-50.
76. Seroogy, K.B., et.al. 1994. J Comp Neurol. 342, 321-34.
77. Skaper, S.D., 2012. Methods Mol Biol. 846, 1-12.
78. Somoza, R., et.al. 2010. Biol Blood Marrow Transplant. 16, 1530-40.
79. Spina, M.B., et.al. 1992. J Neurochem. 59, 99-106.
80. Suarez, C.E., et.al. 2006. Int J Parasitol. 36, 965-73.
81. Sun, M., et.al. 2005. Brain Res. 1052, 119-29.
82. Szigethy, et.al. 1989. J Comp Neurol. 279, 128-37.
83. Tucker, K., et.al. 2002. J Physiol. 542, 413-29.
84. Ugrumov, M.V., et.al. 2011. Neuroscience. 181, 175-88.
85. Uhl, G.R., et.al. 1984. Brain Res. 308, 186-90.
86. Vanitallie, T.B. 2008. Metabolism. 57 Suppl 2, S50-5.
87. Venero, J.L., et.al. 2000. Exp Neurol. 161, 38-48.
88. Wakabayashi-Ito, N., et.al. 1994. J Biol Chem. 269, 29831-7.
89. Ward, N.L., et.al. 2000. Exp Neurol. 162, 297-310.
90. Yamada, M., et.al. 1995. Neuroscience. 64, 405-17.
91. Yoshimoto, Y., et.al. 1995. Brain Res. 691, 25-36.
92. Yurek, D.M., et.al. 1996. Exp Neurol. 137, 105-18.
93. Zamudio, S., et.al. 2005. Brain Res Bull. 65, 339-47.
94. Zhou, J., et.al. 1996. Brain Res Dev Brain Res. 97, 297-303.
95. Zhou, J., et.al. 1997. Brain Res Dev Brain Res. 100, 43-51.

## Claims

1. Nanomolecular complex NTS-polyplex comprising poly-L-lysine, neurotensin (NTS), a fusogenic peptide, a caryophillic peptide and a plasmid encoding the BDNF gene, which expression is controlled by the hDAT promoter wherein the plasmid is phDAT-BDNF-flag, for use in a treating of Parkinson's disease.

2. NTS-polyplex for use according to claim 1, wherein the fusogenic peptide is derived from H2A hemagglutinin influenza virus.

3. NTS-polyplex for use according to claim 1, wherein the treatment of Parkinson's disease is preventive of the manifestation of symptoms, and where there still are 50 to 60% of neurons in the substantia nigra and 20% of dopaminergic innervation in the putamen.

4. NTS-polyplex for use according to claim 1, wherein the treatment of Parkinson's disease is to decrease the symptoms, and wherein the patient shows decreased striatal dopamine levels up to 80%.

5. A pharmaceutical composition for use in the treating of Parkinson's disease comprising a NTS-polyplex nanomolecular complex comprising poly-L-lysine, neurotensin (NTS), a fusogenic peptide, a caryophillic peptide and a plasmid encoding the BDNF gene, which expression is controlled by the hDAT promoter wherein the plasmid is phDAT-BDNF-flag; and a pharmaceutically acceptable carrier.

6. The pharmaceutical composition for use according to claim 5, wherein the plasmid is phDAT-BDNF-flag.

7. The pharmaceutical composition for use according to claim 5, wherein the fusogenic peptide is derived of H2A hemagglutinin influenza virus.

## Patentansprüche

1. Nanomolekularer komplexer NTS-Polyplex, der umfasst: Poly-L-Lysin, Neurotensin (NTS), ein fusogenes Peptid, ein caryophiles Peptid und ein Plasmid, das das BDNF-Gen kodiert, dessen Expression durch den hDAT-Promoter gesteuert wird, wobei das Plasmid phDAT-BDNF-Flag ist, zur Verwendung bei einer Behandlung von Morbus Parkinson.

2. NTS-Polyplex zur Verwendung gemäß Anspruch 1, wobei das fusogene Peptid von dem H2A-Hämagglutinin-Influenzavirus erhalten wird.

3. NTS-Polyplex zur Verwendung gemäß Anspruch 1, wobei die Behandlung von Morbus Parkinson vor der Manifestation von Symptomen schützt und es noch 50 - 60 % von Neuronen in der Substantia nigra und 20 % einer dopaminergen Innervation im Putamen gibt.

4. NTS-Polyplex zur Verwendung gemäß Anspruch 1, wobei die Behandlung von Morbus Parkinson darin besteht, die Symptome zu verringern, und wobei der Patient verringerte striatale Dopaminpegel bis zu 80 % zeigt.

5. Pharmazeutische Zusammensetzung zur Verwendung bei der Behandlung von Morbus Parkinson, die umfasst: einen nanomolekularen NTS-Polyplex-Komplex, der umfasst: Poly-L-Lysin, Neurotensin (NTS), ein fusogenes Peptid, ein caryophiles Peptid und ein Plasmid, das das BDNF-Gen kodiert, dessen Expression durch den hDAT-Promoter gesteuert wird, wobei das Plasmid phDAT-BDNF-Flag ist; und einen pharmazeutisch akzeptablen Träger.

6. Pharmazeutische Zusammensetzung zur Verwendung gemäß Anspruch 5, wobei das Plasmid phDAT-BDNF-Flag ist.

7. Pharmazeutische Zusammensetzung zur Verwendung gemäß Anspruch 5, wobei das fusogene Peptid von dem H2A-Hämagglutinin-Influenzavirus erhalten wird.

## Revendications

1. Complexe nanomoléculaire NTS-polyplex comprenant une poly-L-lysine, une neurotensine (NTS), un peptide fusogène, un peptide caryophile et un plasmide codant pour le gène BDNF, dont l'expression est régulée par le promoteur hDAT, où le plasmide est phDAT-BDNF-flag, pour son utilisation dans le traitement de la maladie de Parkinson.

2. NTS-polyplex pour son utilisation selon la revendication 1, dans lequel le peptide fusogène est dérivé de l'hémagglutinine H2A du virus influenza.

3. NTS-polyplex pour son utilisation selon la revendication 1, dans lequel le traitement de la maladie de Parkinson prévient la manifestation des symptômes, et où il subsiste encore de 50 à 60 % de neurones dans la substance noire et 20 % d'innervation dopaminergique dans le putamen.

4. NTS-polyplex pour son utilisation selon la revendication 1, dans lequel le traitement de la maladie de Parkinson vise à réduire les symptômes, et dans lequel le patient présente des déficits de niveaux de dopamine striale jusqu'à 80 %.

5. Composition pharmaceutique pour son utilisation dans le traitement de la maladie de Parkinson comprenant un complexe nanomoléculaire NTS-polyplex comprenant une poly-L-lysine, une neurotensine (NTS), un peptide fusogène, un peptide caryophile et un plasmide codant pour le gène BDNF, dont l'expression est régulée par le promoteur hDAT, où le plasmide est phDAT-BDNF-flag ; et un véhicule pharmaceutiquement acceptable.

6. Composition pharmaceutique pour son utilisation selon la revendication 5, dans laquelle le plasmide est phDAT-BDNF-flag.

7. Composition pharmaceutique pour son utilisation selon la revendication 5, dans laquelle le plasmide fusogène est dérivé de l'hémagglutinine H2A du virus influenza.
